# EUROPEAN PATENT SPECIFICATION

(11) **EP 3 831 421 B1**
(45) Date of publication and mention of the grant of the patent: **10.06.2026**
(21) Application number: 19857408.9
(22) Date of filing: 29.08.2019
(51) Int. Cl.: A61M 1/36

(54) **EXTRACORPOREAL CIRCULATION MANAGEMENT DEVICE, EXTRACORPOREAL CIRCULATION DEVICE, AND EXTRACORPOREAL CIRCULATION MANAGEMENT PROGRAM**
VORRICHTUNG ZUR VERWALTUNG DER EXTRAKORPORALEN ZIRKULATION, VORRICHTUNG ZUR EXTRAKORPORALEN ZIRKULATION UND PROGRAMM ZUR VERWALTUNG DER EXTRAKORPORALEN ZIRKULATION
DISPOSITIF DE GESTION DE CIRCULATION EXTRACORPORELLE, DISPOSITIF DE CIRCULATION EXTRACORPORELLE ET PROGRAMME DE GESTION DE CIRCULATION EXTRACORPORELLE

(30) Priority: 06.09.2018 JP 2018167079
(43) Date of publication of application: 09.06.2021
(73) Proprietor: TERUMO Kabushiki Kaisha, Tokyo 151-0072 (JP)
(72) Inventor: HASHIMOTO, Tomoaki, Ashigarakami-gun Kanagawa 259-0151 (JP)
(74) Representative: Casalonga
(86) International application number: PCT/JP2019/033967
(87) International publication number: WO 2020/050136

(56) References cited:
- WO-A1-2016/035714
- WO-A1-2016/092913
- WO-A1-2016/092913
- WO-A1-2019/181610
- WO-A2-02/26286
- JP-A- 2017 038 805
- US-A1- 2013 094 996
- KOYAMA TOMIO : "Usefulness of displaying 'flow rate-pressure drop' of artificial lung pressure in real time", JAPANESE JOURNAL OF EXTRA-CORPOREAL TECHNOLOGY, vol. 44, no. 3, 2017, pages 288, XP009526140, ISSN: 0912-2664

## Description

### Technical Field

The present invention relates to an extracorporeal circulation management device, an extracorporeal circulation device, and an extracorporeal circulation management program.

### Background Art

For example, when it is necessary to supply blood to a patient during a surgery, extracorporeal circulation is performed in which the patient's blood is circulated extracorporeally using an extracorporeal circulation device having an artificial heart-lung device or the like. In addition, there are various auxiliary circulation methods, such as veno-arterial extracorporeal membrane oxygenation (VA-ECMO) and veno-venous extracorporeal membrane oxygenation (VV-ECMO), as long-term life support by extracorporeal circulation of blood.

For example, in auxiliary circulation techniques using VV-ECMO, extracorporeal circulation has been increasingly performed for a long period of time. Therefore, it becomes important not only to manage a state of a patient but also to grasp states of devices such as circulation circuits, pumps, and oxygenators, used for extracorporeal circulation such that extracorporeal circulation can be appropriately performed.

In addition, devices, such as pressure sensors and flow rate sensors, used for extracorporeal circulation have increased. Accordingly, the number of items to be monitored in the auxiliary circulation techniques has increased. Therefore, it is required to understand the meaning of values measured by the respective devices and to appropriately grasp the values measured by the respective devices in the medical field. For example, it is important to grasp a distribution and a state of a pressure applied in a circulation circuit in the medical field. If a blood removal pressure (for example, negative pressure) increases when a flow rate of blood flowing through the circulation circuit decreases, blood removal failure is suspected in the medical field based on a rule of thumb of an operator or the like. Further, the operator or the like searches for a cause of the blood removal failure in a circuit or device on the blood removal side, and resolves the blood removal failure.

Patent Literature 1 discloses an extracorporeal circulation management device that displays state information of a pressure sensor, a flow rate sensor, and the like on a display unit as continuous state information over time, and displays warning information on the display unit based on trend information of the continuous state information over time. In the extracorporeal circulation management device described in Patent Literature 1, however, there is room for improvement in that it is difficult for an operator to easily grasp a cause of circulation failure in extracorporeal circulation although the continuous state information over time and the warning information are displayed on the display unit.

### Citation List

### Patent Literature

Patent Literature 1: JP 2017-38805 A

WO0226286A2 provides a further extracorporeal circulation management device configured to manage an extracorporeal circulation device extracorporeally circulating blood using a circulation circuit.

### Summary of Invention

### Technical Problem

The present invention has been made to solve the above problem, and an object thereof is to provide an extracorporeal circulation management device, an extracorporeal circulation device, and an extracorporeal circulation management program that allow an operator or like to easily grasp a cause of circulation failure in extracorporeal circulation.

### Solution to Problem

According to the present invention, the above problem is solved by an extracorporeal circulation management device according to independent claim 1. The dependent claims relate to advantageous embodiments. Independent claim 7 defines an extracorporeal circulation device according to the invention and independent claim 8 defines an extracorporeal circulation management program according to the invention.

According to the extracorporeal circulation management device of the present invention, the assumed flow rate assumed in advance as the assumed value of the flow rate of the blood flowing inside the circulation circuit and the actual flow rate measured by the flow rate measurement unit as the actually measured value of the flow rate of the blood flowing inside the circulation circuit are displayed on the display unit. As a result, an operator and the like can easily grasp a discrepancy or deviation between the assumed flow rate of the blood in the circulation circuit and the actual flow rate of the blood actually flowing through the circulation circuit by confirming the display unit. In addition, the standard pressure calculated based on the assumed flow rate by referring to the standard information stored in the storage unit, which is the standard information representing the relation between the blood flow rate and the pressure loss occurring in the device provided in the circulation circuit, and the actual pressure related to the device, calculated based on the actual pressure measured by the pressure measurement unit as the actually measured value of the pressure of the blood flowing inside the circulation circuit and the actual flow rate measured by the flow rate measurement unit, are displayed on the display unit. As a result, the operator or the like can easily grasp a discrepancy or deviation between the standard pressure calculated based on the assumed flow rate and the actual pressure related to the device by confirming the display unit. Therefore, when there is a discrepancy or deviation between the assumed flow rate and the actual flow rate, the operator or the like can easily grasp the occurrence of the discrepancy or deviation between the assumed flow rate and the actual flow rate and grasp a location of the circulation circuit where the discrepancy or deviation between the standard pressure and the actual pressure has occurred and easily grasp that a cause of circulation failure exists near the location by confirming the display unit. That is, the location of the circulation circuit where the discrepancy or deviation between the standard pressure and the actual pressure has occurred corresponds to a portion where the pressure measurement unit is provided or a portion of the device provided near the pressure measurement unit. In this manner, the operator or the like can easily grasp the cause of the circulation failure in the extracorporeal circulation.

Preferably, the extracorporeal circulation management device according to the present invention further displays a differential flow rate representing a difference between the assumed flow rate and the actual flow rate and a differential pressure representing a difference between the standard pressure and the actual pressure related to the device on the display unit.

According to a preferred embodiment of the extracorporeal circulation management device of the present invention, the differential flow rate representing the difference between the assumed flow rate and the actual flow rate and the differential pressure representing the difference between the standard pressure and the actual pressure related to the device are further displayed on the display unit. As a result, the operator or the like can more easily grasp a discrepancy or deviation between the assumed flow rate and the actual flow rate and a discrepancy or deviation between the standard pressure and the actual pressure by confirming the display unit. The standard pressure is calculated based on the standard information stored in the storage unit and the assumed flow rate. That is, the standard pressure changes depending on the assumed flow rate. Therefore, a discrepancy or deviation sometimes occurs between the standard pressure and the actual pressure even if the actual pressure related to the device does not change at first glance. On the other hand, the operator or the like can more easily grasp the discrepancy or deviation between the standard pressure and the actual pressure by confirming the display unit according to the extracorporeal circulation management device of the present invention.

Preferably, in the extracorporeal circulation management device according to the present invention, the device includes a plurality of instrument elements, the pressure measurement unit includes a plurality of pressure sensors. A plurality of the standard pressures calculated based on the assumed flow rate by referring to a plurality of pieces of the standard information each representing a relation between the blood flow rate and the pressure loss occurring in each of the plurality of instrument elements and stored in the storage unit, and the actual pressures related to the plurality of instrument elements, calculated based on a plurality of the actual pressures measured by the plurality of pressure sensors and the actual flow rate, are displayed on the display unit. A plurality of the differential pressures each representing a difference between each of the plurality of standard pressures and each of the actual pressures related to the plurality of instrument elements are displayed on the display unit.

According to a preferred embodiment of the extracorporeal circulation management device of the present invention, the plurality of standard pressures calculated based on the assumed flow rate by referring to the plurality of pieces of standard information stored in the storage unit, which are the plurality of pieces of standard information each representing the relation between the blood flow rate and the pressure loss occurring in each of the plurality of instrument elements, and the actual pressures related to the plurality of instrument elements, calculated based on the plurality of actual pressures measured by the plurality of pressure sensors and the actual flow rate measured by the flow rate measurement unit, are displayed on the display unit. In addition, the plurality of differential pressures each representing the difference between each of the plurality of standard pressures and each of the actual pressures related to the plurality of instrument elements are displayed on the display unit. Therefore, the operator or the like can more easily grasp a discrepancy or deviation between each of the plurality of standard pressures and each of the actual pressures related to the plurality of instrument elements and easily grasp a location of the circulation circuit where the discrepancy or deviation between the standard pressure and the actual pressure has occurred from among a plurality of locations and easily grasp that a cause of circulation failure exists near the location by confirming the display unit. That is, the location of the circulation circuit where the discrepancy or deviation between the standard pressure and the actual pressure has occurred corresponds to a portion where a pressure sensor detecting an abnormal value among the plurality of pressure sensors is provided or a portion of an instrument element provided near the pressure sensor detecting the abnormal value among the plurality of instrument elements.

Preferably, in the extracorporeal circulation management device according to the present invention, the plurality of instrument elements include: a blood removing catheter configured to be partially inserted into a patient and guide the blood taken out from the patient; a pump configured to be provided on a downstream side of the blood removing catheter, take out the blood from the patient through the blood removing catheter and send the blood to the downstream side; an oxygenator configured to be provided on the downstream side of the pump and perform a gas exchange operation for the blood; and a blood feeding catheter configured to be provided on the downstream side of the oxygenator and be partially inserted into the patient, and guide the blood that has passed through the oxygenator to the patient. The plurality of pressure sensors include: a first pressure sensor configured to be provided in the circulation circuit at least between the blood removing catheter and the pump or between the pump and the oxygenator; and a second pressure sensor configured to be provided in the circulation circuit between the oxygenator and the blood feeding catheter. The flow rate measurement unit is a flow rate sensor provided in the circulation circuit. All the assumed flow rate, the actual flow rate acquired by the flow rate sensor, the differential flow rate, the standard pressure related to the blood removing catheter calculated based on the assumed flow rate by referring to the standard information related to the blood removing catheter, which represents a relation between the blood flow rate and the pressure loss occurring in the blood removing catheter and is stored in the storage unit, the standard pressure related to the oxygenator calculated based on the assumed flow rate by referring to the standard information related to the oxygenator, which represents a relation between the blood flow rate and the pressure loss occurring in the oxygenator and is stored in the storage unit, the standard pressure related to the blood feeding catheter calculated based on the assumed flow rate by referring to the standard information related to the blood feeding catheter, which represents a relation between the blood flow rate and the pressure loss occurring in the blood feeding catheter and is stored in the storage unit, the actual pressure related to the blood removing catheter calculated based on the actual pressure measured by the first pressure sensor and the actual flow rate, the actual pressure related to the oxygenator calculated based on the actual pressure measured by the first pressure sensor, the actual pressure measured by the second pressure sensor, and the actual flow rate, the actual pressure related to the blood feeding catheter calculated based on the actual pressure measured by the second pressure sensor and the actual flow rate, the differential pressure related to the blood removing catheter which represents a difference between the standard pressure related to the blood removing catheter and the actual pressure related to the blood removing catheter, the differential pressure related to the oxygenator which represents a difference between the standard pressure related to the oxygenator and the actual pressure related to the oxygenator, and the differential pressure related to the blood feeding catheter which represents a difference between the standard pressure related to the blood feeding catheter and the actual pressure related to the blood feeding catheter are simultaneously displayed on the display unit.

According to a preferred embodiment of the extracorporeal circulation management device of the present invention, the standard pressure related to the blood removing catheter calculated based on the assumed flow rate by referring to the standard information related to the blood removing catheter stored in the storage unit, which is the standard information related to the blood removing catheter representing the relation between the blood flow rate and the pressure loss occurring in the blood removing catheter, is displayed on the display unit. In addition, the standard pressure related to the oxygenator calculated based on the assumed flow rate by referring to the standard information related to the oxygenator stored in the storage unit, which is the standard information related to the oxygenator representing the relation between the blood flow rate and the pressure loss occurring in the oxygenator, is displayed on the display unit. In addition, the standard pressure related to the blood feeding catheter calculated based on the assumed flow rate by referring to the standard information related to the blood feeding catheter stored in the storage unit, which is the standard information related to the blood feeding catheter representing the relation between the blood flow rate and the pressure loss occurring in the blood feeding catheter, is displayed on the display unit. In addition, the actual pressure related to the blood removing catheter, calculated based on the actual pressure measured by the first pressure sensor and the actual flow rate acquired by the flow rate sensor, is displayed on the display unit. In addition, the actual pressure related to the oxygenator, calculated based on the actual pressure measured by the first pressure sensor, the actual pressure measured by the second pressure sensor, and the actual flow rate acquired by the flow rate sensor, is displayed on the display unit. In addition, the actual pressure related to the blood feeding catheter, calculated based on the actual pressure measured by the second pressure sensor and the actual flow rate acquired by the flow rate sensor, is displayed on the display unit. In addition, the differential pressure related to the blood removing catheter, which represents the difference between the standard pressure related to the blood removing catheter and the previous pressure related to the blood removing catheter, is displayed on the display unit. In addition, the differential pressure related to the oxygenator, which represents the difference between the standard pressure related to the oxygenator and the actual pressure related to the oxygenator, is displayed on the display unit. In addition, the differential pressure related to the blood feeding catheter, which represents the difference between the standard pressure related to the blood feeding catheter and the actual pressure related to the blood feeding catheter, is displayed on the display unit. Further, all the assumed flow rate, the actual flow rate, the differential flow rate, the standard pressure related to the blood removing catheter, the standard pressure related to the oxygenator, the standard pressure related to the blood feeding catheter, the actual pressure related to the blood removing catheter, the actual pressure related to the oxygenator, the actual pressure related to the blood feeding catheter, the differential pressure related to the blood removing catheter, the differential pressure related to the oxygenator, and the differential pressure related to the blood feeding catheter are simultaneously displayed on the display unit. As a result, the operator or the like can easily grasp a discrepancy or deviation between the standard pressure and the actual pressure and more concretely and easily grasp a location of the circulation circuit where the discrepancy or deviation between the standard pressure and the actual pressure has occurred and easily grasp that a cause of circulation failure exists near the location by confirming the display unit. That is, the location of the circulation circuit where the discrepancy or deviation between the standard pressure and the actual pressure has occurred corresponds to a portion where a pressure sensor detecting an abnormal value between the first pressure sensor and the second pressure sensor is provided or a portion of an instrument element provided near the pressure sensor detecting the abnormal value among the blood removing catheter, the oxygenator, and the blood feeding catheter.

Preferably, in the extracorporeal circulation management device according to the present invention, the plurality of instrument elements include: a blood removing catheter which is partially inserted into a patient and guides the blood taken out from the patient; a pump which is provided on a downstream side of the blood removing catheter, takes out the blood from the patient through the blood removing catheter, and sends the blood to the downstream side; an oxygenator which is provided on the downstream side of the pump and performs a gas exchange operation for the blood; and a blood feeding catheter which is provided on the downstream side of the oxygenator and is partially inserted into the patient, and guides the blood that has passed through the oxygenator to the patient. The plurality of pressure sensors include: a first pressure sensor provided in the circulation circuit between the blood removing catheter and the pump; a second pressure sensor provided in the circulation circuit between the pump and the oxygenator; and a third pressure sensor provided in the circulation circuit between the oxygenator and the blood feeding catheter. The flow rate measurement unit is a flow rate sensor provided in the circulation circuit. All the assumed flow rate, the actual flow rate acquired by the flow rate sensor, the differential flow rate, the standard pressure related to the blood removing catheter calculated based on the assumed flow rate by referring to the standard information related to the blood removing catheter which represents a relation between the blood flow rate and the pressure loss occurring in the blood removing catheter and is stored in the storage unit, the standard pressure related to the oxygenator calculated based on the assumed flow rate by referring to the standard information related to the oxygenator which represents a relation between the blood flow rate and the pressure loss occurring in the oxygenator and is stored in the storage unit, the standard pressure related to the blood feeding catheter calculated based on the assumed flow rate by referring to the standard information related to the blood feeding catheter which represents a relation between the blood flow rate and the pressure loss occurring in the blood feeding catheter and is stored in the storage unit, the actual pressure related to the blood removing catheter calculated based on the actual pressure measured by the first pressure sensor and the actual flow rate, the actual pressure related to the oxygenator calculated based on the actual pressure measured by the second pressure sensor, the actual pressure measured by the third pressure sensor, and the actual flow rate, the actual pressure related to the blood feeding catheter calculated based on the actual pressure measured by the third pressure sensor and the actual flow rate, the differential pressure related to the blood removing catheter which represents a difference between the standard pressure related to the blood removing catheter and the actual pressure related to the blood removing catheter, the differential pressure related to the oxygenator which represents a difference between the standard pressure related to the oxygenator and the actual pressure related to the oxygenator, and the differential pressure related to the blood feeding catheter which represents a difference between the standard pressure related to the blood feeding catheter and the actual pressure related to the blood feeding catheter are simultaneously displayed on the display unit.

According to a preferred embodiment of the extracorporeal circulation management device of the present invention, the standard pressure related to the blood removing catheter calculated based on the assumed flow rate by referring to the standard information related to the blood removing catheter stored in the storage unit, which is the standard information related to the blood removing catheter representing the relation between the blood flow rate and the pressure loss occurring in the blood removing catheter, is displayed on the display unit. In addition, the standard pressure related to the oxygenator calculated based on the assumed flow rate by referring to the standard information related to the oxygenator stored in the storage unit, which is the standard information related to the oxygenator representing the relation between the blood flow rate and the pressure loss occurring in the oxygenator, is displayed on the display unit. In addition, the standard pressure related to the blood feeding catheter calculated based on the assumed flow rate by referring to the standard information related to the blood feeding catheter stored in the storage unit, which is the standard information related to the blood feeding catheter representing the relation between the blood flow rate and the pressure loss occurring in the blood feeding catheter, is displayed on the display unit. In addition, the actual pressure related to the blood removing catheter, calculated based on the actual pressure measured by the first pressure sensor and the actual flow rate acquired by the flow rate sensor, is displayed on the display unit. In addition, the actual pressure related to the oxygenator, calculated based on the actual pressure measured by the second pressure sensor, the actual pressure measured by the third pressure sensor, and the actual flow rate acquired by the flow rate sensor, is displayed on the display unit. In addition, the actual pressure related to the blood feeding catheter, calculated based on the actual pressure measured by the third pressure sensor and the actual flow rate acquired by the flow rate sensor, is displayed on the display unit. In addition, the differential pressure related to the blood removing catheter, which represents the difference between the standard pressure related to the blood removing catheter and the actual pressure related to the blood removing catheter, is displayed on the display unit. In addition, the differential pressure related to the oxygenator, which represents the difference between the standard pressure related to the oxygenator and the actual pressure related to the oxygenator, is displayed on the display unit. In addition, the differential pressure related to the blood feeding catheter, which represents the difference between the standard pressure related to the blood feeding catheter and the actual pressure related to the blood feeding catheter, is displayed on the display unit. Further, all the assumed flow rate, the actual flow rate, the differential flow rate, the standard pressure related to the blood removing catheter, the standard pressure related to the oxygenator, the standard pressure related to the blood feeding catheter, the actual pressure related to the blood removing catheter, the actual pressure related to the oxygenator, the actual pressure related to the blood feeding catheter, the differential pressure related to the blood removing catheter, the differential pressure related to the oxygenator, and the differential pressure related to the blood feeding catheter are simultaneously displayed on the display unit. As a result, the operator or the like can easily grasp a discrepancy or deviation between the standard pressure and the actual pressure and more concretely and easily grasp a location of the circulation circuit where the discrepancy or deviation between the standard pressure and the actual pressure has occurred and easily grasp that a cause of circulation failure exists near the location by confirming the display unit. That is, the location of the circulation circuit where the discrepancy or deviation between the standard pressure and the actual pressure has occurred corresponds to a portion where a pressure sensor detecting an abnormal value among the first pressure sensor, the second pressure sensor, and the third pressure sensor is provided or a portion of an instrument element provided near the pressure sensor detecting the abnormal value among the blood removing catheter, the oxygenator, and the blood feeding catheter.

Preferably, the extracorporeal circulation management device according to the present invention provides notification of a warning when an absolute value of at least one of the differential flow rate and the differential pressure exceeds a predetermined value.

According to a preferred embodiment of the extracorporeal circulation management device of the present invention, the operator or the like can more easily grasp occurrence of a discrepancy or deviation between the assumed flow rate and the actual flow rate and further grasp a location of the circulation circuit where the discrepancy or deviation between the standard pressure and the actual pressure has occurred and more easily grasp that a cause of circulation failure exists near the location.

According to the present invention, the above problem is solved by an extracorporeal circulation device which extracorporeally circulates blood using a circulation circuit, the extracorporeal circulation device including: a display unit that displays various types of information; a blood removing catheter which is partially inserted into a patient and guides the blood taken out from the patient; a pump which is provided on a downstream side of the blood removing catheter, takes out the blood from the patient through the blood removing catheter, and sends the blood to the downstream side; an oxygenator which is provided on the downstream side of the pump and performs a gas exchange operation for the blood; a blood feeding catheter which is provided on the downstream side of the oxygenator, is partially inserted into the patient, and guides the blood that has passed through the oxygenator to the patient; and any of the above-described extracorporeal circulation management devices.

According to the extracorporeal circulation device of the present invention, the assumed flow rate assumed in advance as the assumed value of the flow rate of the blood flowing inside the circulation circuit and the actual flow rate measured by the flow rate measurement unit as the actually measured value of the flow rate of the blood flowing inside the circulation circuit are displayed on the display unit. As a result, an operator and the like can easily grasp a discrepancy or deviation between the assumed flow rate of the blood in the circulation circuit and the actual flow rate of the blood actually flowing through the circulation circuit by confirming the display unit. In addition, the standard pressure calculated based on the assumed flow rate by referring to the standard information stored in the storage unit, which is the standard information representing the relation between the blood flow rate and the pressure loss occurring in the device, such as the blood removing catheter, the pump, the oxygenator, and the blood feeding catheter, provided in the circulation circuit, and the actual pressure related to the device, calculated based on the actual pressure measured by the pressure measurement unit as the actually measured value of the pressure of the blood flowing inside the circulation circuit and the actual flow rate measured by the flow rate measurement unit, are displayed on the display unit. As a result, the operator or the like can easily grasp a discrepancy or deviation between the standard pressure calculated based on the assumed flow rate and the actual pressure related to the device by confirming the display unit. Therefore, when there is a discrepancy or deviation between the assumed flow rate and the actual flow rate, the operator or the like can easily grasp the occurrence of the discrepancy or deviation between the assumed flow rate and the actual flow rate and grasp a location of the circulation circuit where the discrepancy or deviation between the standard pressure and the actual pressure has occurred and easily grasp that a cause of circulation failure exists near the location by confirming the display unit. That is, the location of the circulation circuit where the discrepancy or deviation between the standard pressure and the actual pressure has occurred corresponds to a portion where the pressure measurement unit is provided or a portion of the device provided near the pressure measurement unit. In this manner, the operator or the like can easily grasp the cause of the circulation failure in the extracorporeal circulation.

According to the present invention, the above problem is solved by an extracorporeal circulation management program executed by a computer of an extracorporeal circulation management device that manages an extracorporeal circulation device which extracorporeally circulates blood using a circulation circuit, the extracorporeal circulation management program causing the computer to execute a step of displaying an assumed flow rate, which is assumed in advance as an assumed value of a flow rate of the blood flowing inside the circulation circuit, and an actual flow rate, which is measured by a flow rate measurement unit as an actually measured value of the flow rate of the blood flowing inside the circulation circuit, on a display unit, and displaying a standard pressure calculated based on the assumed flow rate by referring to standard information, which represents a relation between the blood flow rate and a pressure loss occurring in a device provided in the circulation circuit and is stored in a storage unit, and an actual pressure related to the device, which is calculated based on an actual pressure measured by a pressure measurement unit as an actually measured value of a pressure of the blood flowing inside the circulation circuit and the actual flow rate, on the display unit.

According to the extracorporeal circulation management program of the present invention, the assumed flow rate assumed in advance as the assumed value of the flow rate of the blood flowing inside the circulation circuit and the actual flow rate measured by the flow rate measurement unit as the actually measured value of the flow rate of the blood flowing inside the circulation circuit are displayed on the display unit. As a result, an operator and the like can easily grasp a discrepancy or deviation between the assumed flow rate of the blood in the circulation circuit and the actual flow rate of the blood actually flowing through the circulation circuit by confirming the display unit. In addition, the standard pressure calculated based on the assumed flow rate by referring to the standard information stored in the storage unit, which is the standard information representing the relation between the blood flow rate and the pressure loss occurring in the device provided in the circulation circuit, and the actual pressure related to the device, calculated based on the actual pressure measured by the pressure measurement unit as the actually measured value of the pressure of the blood flowing inside the circulation circuit and the actual flow rate measured by the flow rate measurement unit, are displayed on the display unit. As a result, the operator or the like can easily grasp a discrepancy or deviation between the standard pressure calculated based on the assumed flow rate and the actual pressure related to the device by confirming the display unit. Therefore, when there is a discrepancy or deviation between the assumed flow rate and the actual flow rate, the operator or the like can easily grasp the occurrence of the discrepancy or deviation between the assumed flow rate and the actual flow rate and grasp a location of the circulation circuit where the discrepancy or deviation between the standard pressure and the actual pressure has occurred and easily grasp that a cause of circulation failure exists near the location by confirming the display unit. That is, the location of the circulation circuit where the discrepancy or deviation between the standard pressure and the actual pressure has occurred corresponds to a portion where the pressure measurement unit is provided or a portion of the device provided near the pressure measurement unit. In this manner, the operator or the like can easily grasp the cause of the circulation failure in the extracorporeal circulation.

### Advantageous Effects of Invention

According to the present invention, it is possible to provide the extracorporeal circulation management device, the extracorporeal circulation device, and the extracorporeal circulation management program that allow the operator or the like to easily grasp the cause of the circulation failure in the extracorporeal circulation.

### Brief Description of Drawings

Fig. 1 is a schematic diagram illustrating an extracorporeal circulation device according to an embodiment of the present invention.
Fig. 2 is a schematic diagram illustrating an extracorporeal circulation device according to a modification of the present embodiment.
Fig. 3 is a block diagram illustrating a main configuration of an extracorporeal circulation management device according to the present embodiment.
Fig. 4 is a block diagram illustrating a main configuration of the extracorporeal circulation device according to the present embodiment.
Fig. 5 is a schematic diagram illustrating a standard information storage unit of the present embodiment.
Fig. 6 is a graph illustrating an example of standard information related to a blood removing catheter of the present embodiment.
Fig. 7 is a graph illustrating an example of standard information related to an oxygenator of the present embodiment.
Fig. 8 is a graph illustrating an example of standard information related to a blood feeding catheter of the present embodiment.
Fig. 9 is a schematic diagram illustrating a standard pressure calculation unit of the present embodiment.
Fig. 10 is a schematic diagram illustrating an actual pressure calculation unit of the present embodiment.
Fig. 11 is a schematic diagram illustrating a differential pressure calculation unit of the present embodiment.
Fig. 12 is a schematic diagram illustrating a warning information storage unit of the present embodiment.
Fig. 13 is a schematic diagram illustrating an example of an image displayed on an external monitor according to the present embodiment.
Fig. 14 is a schematic diagram illustrating an example of a relation between arrangements of a circulation circuit and each device of the extracorporeal circulation device according to the present embodiment and a pressure loss.
Fig. 15 is a schematic diagram illustrating another example of an image displayed on an external monitor of the present embodiment.
Fig. 16 is a flowchart illustrating an example of control executed by a computer of the extracorporeal circulation management device according to the present embodiment.
Fig. 17 is a flowchart illustrating an example of the control executed by the computer of the extracorporeal circulation management device according to the present embodiment.

Description of Embodiments

Hereinafter, preferred embodiments of the present invention will be described in detail with reference to the drawings.

Incidentally, the embodiments to be described below are preferable specific examples of the present invention, and thus, various technically preferable limitations are given. However, a scope of the present invention is not limited to these aspects as long as there is no particular description to limit the present invention in the following description. In addition, the same components will be denoted by the same reference signs in the respective drawings, and the detailed description thereof will be omitted as appropriate.

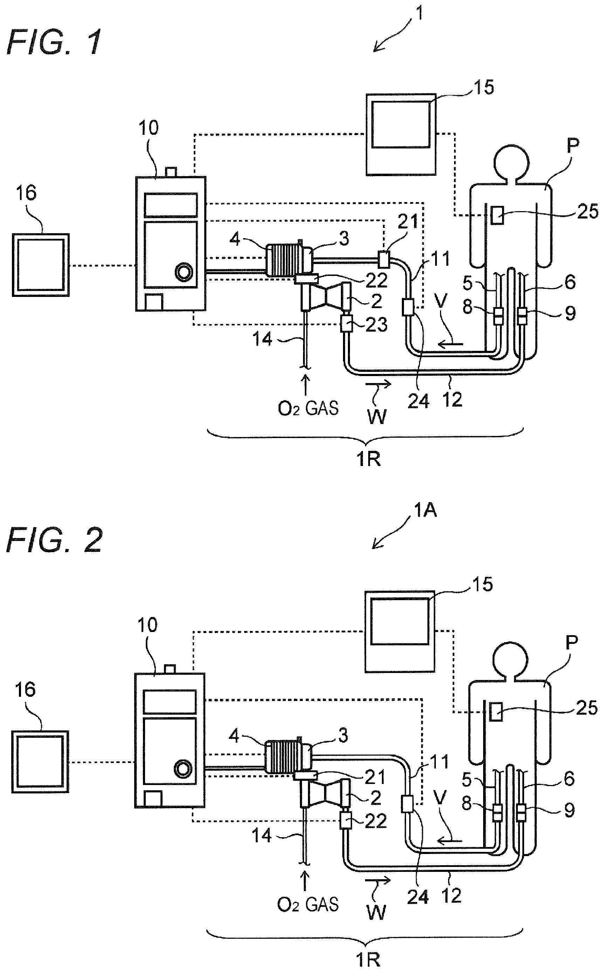

Fig. 1 is a schematic diagram illustrating an extracorporeal circulation device according to an embodiment of the present invention.

Incidentally, an electrical connection is indicated by a broken line in Fig. 1. The electrical connection may be realized by a wire or wirelessly realized.

"Extracorporeal circulation" performed by an extracorporeal circulation device 1 illustrated in Fig. 1 includes an "extracorporeal circulation operation" and an "auxiliary circulation operation". The extracorporeal circulation device 1 can perform both the "extracorporeal circulation operation" and the "auxiliary circulation operation".

The "extracorporeal circulation operation" refers to a blood circulation operation and a gas exchange operation (oxygen addition and/or carbon dioxide removal) for blood performed by the extracorporeal circulation device 1, for example, in a case where blood circulation in the heart is temporarily stopped due to a cardiac surgery.

In addition, the "auxiliary circulation operation" refers to a blood circulation operation and a gas exchange operation for blood that are also performed by the extracorporeal circulation device 1 in a case where the heart of a patient P to which the extracorporeal circulation device 1 is applied, hardly exhibits a sufficient function or in a state where it is difficult to sufficiently perform gas exchange by lungs.

For example, the extracorporeal circulation device 1 is applied in a case where the heart of the patient P does not operate normally or a case where the heart of the patient P operates normally but the lungs do not operate normally. Meanwhile, the extracorporeal circulation device 1 illustrated in Fig. 1 is used, for example, in a case of performing a cardiac surgery on the patient P or in subsequent treatment in an ICU. The extracorporeal circulation device 1 illustrated in Fig. 1 can operate a pump of the extracorporeal circulation device 1 to remove blood from the patient's vein and exchange a gas in the blood using an oxygenator to oxygenate the blood, and then, perform extracorporeal blood circulation of the oxygenator to return the oxygenated blood back to the patient's artery or vein. In this manner, the extracorporeal circulation device 1 is a device that acts as a substitute for the heart and lungs.

As illustrated in Fig. 1, the extracorporeal circulation device 1 has a circulation circuit 1R that circulates blood. The circulation circuit 1R includes an oxygenator 2, a centrifugal pump 3, a drive motor 4 that drives the centrifugal pump 3, a venous catheter (blood removing catheter) 5, an arterial catheter (blood feeding catheter) 6, and an extracorporeal circulation management device 10. The venous catheter 5 is an example of a "blood removing catheter" of the present invention. The arterial catheter 6 is an example of a "blood feeding catheter" of the present invention. The centrifugal pump 3 of the present embodiment is an example of an "instrument element" included in a "device" of the present invention. The extracorporeal circulation management device 10 is provided as a controller of the extracorporeal circulation device 1. Incidentally, the centrifugal pump 3 is also called a blood pump or the like, and may be a pump other than the centrifugal type.

The blood removing catheter 5 is also called a venous cannula (blood removing cannula) and is inserted from the femoral vein. A distal end of the blood removing catheter 5 is placed in the right atrium. The blood removing catheter 5 is connected to a blood removal tube (also referred to as a blood removal line) 11 through a connector 8, is connected to the centrifugal pump 3 through the blood removal tube 11, and guides blood taken out from the patient P to the centrifugal pump 3 through the blood removal tube 11. The blood removal tube 11 is a conduit that connects the blood removing catheter 5 and the centrifugal pump 3, and is the conduit guiding the blood taken out from the patient P through the blood removing catheter 5 to the centrifugal pump 3. The blood removing catheter 5 of the present embodiment is an example of the "instrument element" included in the "device" of the present invention.

The blood feeding catheter 6 is also called an arterial cannula (blood feeding cannula) and is inserted from the femoral artery. The blood feeding catheter 6 is connected to a blood feeding tube (also referred to as a blood feeding line) 12 through the connector 9, is also connected to the oxygenator 2 through the blood feeding tube 12, and guides the blood that has passed through the oxygenator 2 to the patient P through the blood feeding tube 12. The blood feeding tube 12 is a conduit that connects the oxygenator 2 and the blood feeding catheter 6, and is the conduit guiding the blood having passed through the oxygenator 2 to the patient P. The blood feeding catheter 6 of the present embodiment is an example of the "instrument element" included in the "device" of the present invention.

The drive motor 4 controls driving of the centrifugal pump 3 based on a command of the extracorporeal circulation management device 10. The centrifugal pump 3 is provided on the downstream side of the blood removing catheter 5, and is driven by receiving a driving force transmitted from the drive motor 4. The centrifugal pump 3 takes out blood from the patient P through the blood removing catheter 5 and the blood removal tube 11, sends the blood to the oxygenator 2, and then, returns the blood to the patient P through the blood feeding tube 12.

The oxygenator 2 is provided on the downstream side of the centrifugal pump 3. Specifically, the oxygenator 2 is arranged between the centrifugal pump 3 and the blood feeding tube 12. The oxygenator 2 performs the gas exchange operation (oxygen addition and/or carbon dioxide removal) for blood. The oxygenator 2 is, for example, a membrane oxygenator, but is particularly preferably a hollow fiber membrane oxygenator. An oxygen gas is supplied to the oxygenator 2 through an oxygen supply tube 14. The oxygenator 2 of the present embodiment is an example of an "instrument element" included in a "device" of the present invention.

As the blood removal tube 11 and the blood feeding tube 12, for example, a conduit made of a synthetic resin which is highly transparent, elastically deformable, and flexible, such as a vinyl chloride resin and silicone rubber, is used. Blood, which is a liquid, flows in a V direction in the blood removal tube 11 and flows in a W direction in the blood feeding tube 12.

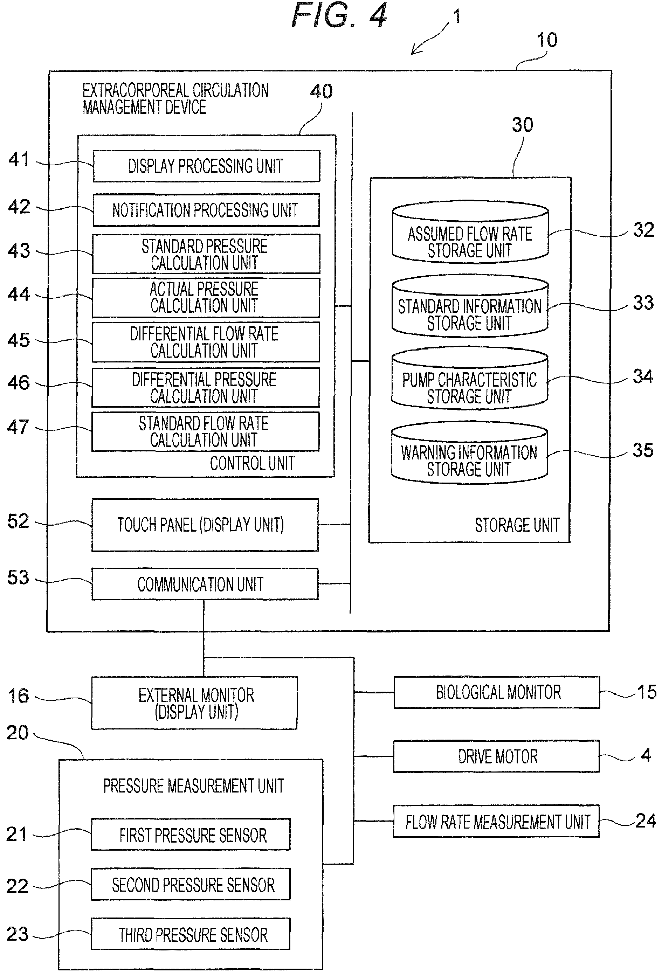

The extracorporeal circulation management device 10 acquires various types of information to perform a calculation, generates a control signal to control operations of devices such as the drive motor 4, a biological monitor 15, and an external monitor 16, and transmits the generated control signal to each device. In other words, the extracorporeal circulation management device 10 manages the extracorporeal circulation device 1. Details of the extracorporeal circulation management device 10 will be described later. In addition, the extracorporeal circulation management device 10 may have a touch panel 52 (see Fig. 4) as an input unit capable of inputting various types of information and as a display unit displaying the various types of information. That is, the "display unit" of the present invention may be the external monitor 16 provided as a separate body from the extracorporeal circulation management device 10, or may be the touch panel 52 provided in the extracorporeal circulation management device 10. The touch panel 52 is capable of detecting contact or the like of a finger of an operator or the like.

The extracorporeal circulation device 1 according to the present embodiment further includes a pressure measurement unit 20 (see Fig. 4), a flow rate measurement unit 24, a blood pressure measurement unit 25, the biological monitor 15, and the external monitor (display unit) 16. The external monitor 16 of the present embodiment is an example of the "display unit" of the present invention. In the following description, a case where the "display unit" of the present invention is the external monitor 16 will be described as an example.

The pressure measurement unit 20 includes a first pressure sensor 21, a second pressure sensor 22, and a third pressure sensor 23. The first pressure sensor 21 is provided in the circulation circuit 1R between the blood removing catheter 5 and the centrifugal pump 3. Specifically, the first pressure sensor 21 is provided on the blood removal tube 11. The first pressure sensor 21 detects a pressure value of blood flowing in the blood removal tube 11. The pressure value detected by the first pressure sensor 21 is an example of an "actual pressure measured by a first pressure sensor" of the present invention. The second pressure sensor 22 is provided in the circulation circuit 1R between the centrifugal pump 3 and the oxygenator 2. The second pressure sensor 22 detects a pressure value of blood flowing inside the circulation circuit 1R between the centrifugal pump 3 and the oxygenator 2. The pressure value detected by the second pressure sensor 22 is an example of an "actual pressure measured by a second pressure sensor" of the present invention. The third pressure sensor 23 is provided in the circulation circuit 1R between the oxygenator 2 and the blood feeding catheter 6. Specifically, the third pressure sensor 23 is provided on the blood feeding tube 12. The third pressure sensor 23 detects a pressure value of blood flowing in the blood feeding tube 12. The pressure value detected by the third pressure sensor 23 is an example of an "actual pressure measured by a third pressure sensor" of the present invention.

The flow rate measurement unit 24 is provided in the circulation circuit 1R between the blood removing catheter 5 and the oxygenator 2. Specifically, the flow rate measurement unit 24 is provided on the blood removal tube 11. Incidentally, the installation position of the flow rate measurement unit 24 is not limited to the blood removal tube 11, and may be any location in the circulation circuit 1R. The flow rate measurement unit 24 is, for example, a flow rate sensor, and detects a value of a flow rate of blood flowing inside the circulation circuit 1R. The value of the flow rate detected by the flow rate measurement unit 24 is an example of an "actual flow rate measured by a flow rate measurement unit" of the present invention.

The blood pressure measurement unit 25 is attached to the patient P and detects a pressure value (blood pressure value) of blood flowing through blood vessels of the patient P. The biological monitor 15 displays the blood pressure and other vital signs of patient P detected by the blood pressure measurement unit 25. The external monitor 16 displays the actual pressure measured by the pressure measurement unit 20 and the actual flow rate measured by the flow rate measurement unit 24 based on the control signal transmitted from the extracorporeal circulation management device 10. Details of an image displayed on the external monitor 16 will be described later.

Fig. 2 is a schematic diagram illustrating an extracorporeal circulation device according to a modification of the present embodiment.

When a component of an extracorporeal circulation device 1A according to the modification illustrated in Fig. 2 is the same as the component of the extracorporeal circulation device 1 according to the present embodiment described with respect to Fig. 1, redundant descriptions will be omitted as appropriate, and differences will be mainly described hereinafter.

In the extracorporeal circulation device 1A according to the modification illustrated in Fig. 2, the first pressure sensor 21 is provided in the circulation circuit 1R between the centrifugal pump 3 and the oxygenator 2. The first pressure sensor 21 of the present modification detects a pressure value of blood flowing inside the circulation circuit 1R between the centrifugal pump 3 and the oxygenator 2. In this case, a pressure value of blood flowing inside the blood removal tube 11 is calculated by subtracting a head of the centrifugal pump 3 from the pressure value detected by the first pressure sensor 21.

The first pressure sensor 21 may be provided in the circulation circuit 1R between the blood removing catheter 5 and the centrifugal pump 3, specifically, in the blood removal tube 11, instead of between the centrifugal pump 3 and the oxygenator 2. In this case, the pressure value of blood flowing inside the circulation circuit 1R between the centrifugal pump 3 and the oxygenator 2 is calculated by adding the head of the centrifugal pump 3 to the pressure value detected by the first pressure sensor 21. In this manner, the first pressure sensor 21 is provided in the circulation circuit 1R at least between the blood removing catheter 5 and the centrifugal pump 3 or between the centrifugal pump 3 and the oxygenator 2 in the extracorporeal circulation device 1A according to the present modification.

The second pressure sensor 22 of the present modification is provided in the circulation circuit 1R between the oxygenator 2 and the blood feeding catheter 6. Specifically, the second pressure sensor 22 is provided on the blood feeding tube 12. The second pressure sensor 22 detects a pressure value of blood flowing in the blood feeding tube 12.

The pressure measurement unit 20 does not necessarily include the first pressure sensor 21, the second pressure sensor 22, and the third pressure sensor 23 as in the extracorporeal circulation device 1A according to the present modification, and can obtain an operation and an effect of the extracorporeal circulation device according to the present embodiment by providing the first pressure sensor 21 and the second pressure sensor 22.

In the following description, the extracorporeal circulation device 1 described above with respect to Fig. 1 will be mainly taken as an example for convenience of the description, and the extracorporeal circulation device 1A illustrated in Fig. 2 will be taken as an example as necessary.

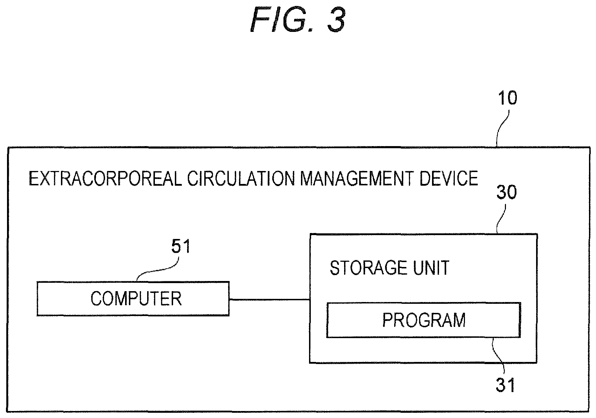

Fig. 3 is a block diagram illustrating a main configuration of an extracorporeal circulation management device according to the present embodiment.

The extracorporeal circulation management device according to the present embodiment includes a computer 51 and a storage unit 30. The computer 51 includes a control unit 40 (see Fig. 4), reads a program 31 stored in the storage unit 30, and executes various calculations and processes. The storage unit 30 stores the program 31 (extracorporeal circulation management program) to be executed by the computer 51. The program 31 of the present embodiment is an example of the "extracorporeal circulation management program" of the present invention. Examples of the storage unit 30 include a hard disk drive (HDD) and the like.

Incidentally, the program 31 is not limited to being stored in the storage unit 30, and may be distributed in a state of being stored in advance in a computer-readable storage medium or may be downloaded to the extracorporeal circulation management device 10 via a network. In addition, the storage unit 30 may be an external storage device connected to the computer 51.

Next, a main configuration of the extracorporeal circulation management device 10 according to the present embodiment will be further described with reference to the drawings.

Fig. 4 is a block diagram illustrating the main configuration of the extracorporeal circulation device according to the present embodiment.

The extracorporeal circulation management device 10 according to the present embodiment includes the control unit 40, the storage unit 30, a touch panel 52, and a communication unit 53. The control unit 40 is, for example, a central processing unit (CPU) or the like, reads the program 31 (see Fig. 3) stored in the storage unit 30, and executes various calculations and processes. The control unit 40 includes a display processing unit 41, a notification processing unit 42, a standard pressure calculation unit 43, an actual pressure calculation unit 44, a differential flow rate calculation unit 45, a differential pressure calculation unit 46, and a standard flow rate calculation unit 47. The display processing unit 41, the notification processing unit 42, the standard pressure calculation unit 43, the actual pressure calculation unit 44, the differential flow rate calculation unit 45, the differential pressure calculation unit 46, and the standard flow rate calculation unit 47 are realized as the computer 51 executes the program 31 stored in the storage unit 30. Incidentally, the display processing unit 41, the notification processing unit 42, the standard pressure calculation unit 43, the actual pressure calculation unit 44, the differential flow rate calculation unit 45, the differential pressure calculation unit 46, and the standard flow rate calculation unit 47 may be realized by hardware, or may be realized by a combination of hardware and software. The storage unit 30 stores the program 31 described above with respect to Fig. 3, and includes an assumed flow rate storage unit 32, a standard information storage unit 33, a pump characteristic storage unit 34, and a warning information storage unit 35.

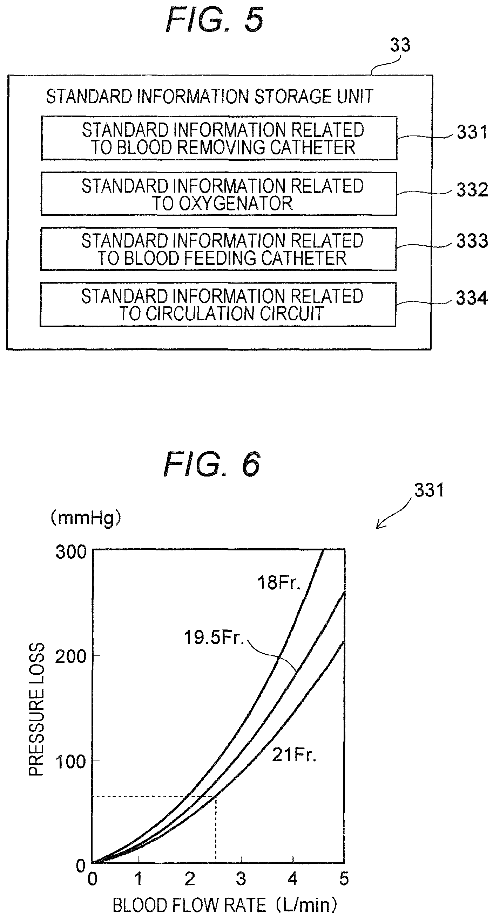

The display processing unit 41 executes a process of displaying an assumed flow rate assumed in advance as an assumed value of a flow rate of blood flowing inside the circulation circuit 1R and an actual flow rate, measured by the flow rate measurement unit 24 as an actually measured value of the flow rate of the blood flowing inside the circulation circuit, on the external monitor 16. In the present embodiment, the "assumed flow rate" is a flow rate value of blood calculated and determined by the standard flow rate calculation unit 47 using, for example, patient information (for example, height, weight, and the like) input by the touch panel 52 in response to the operator's operation on the touch panel 52 and standard information 334 (see Fig. 5) related to the circulation circuit 1R stored in the standard information storage unit 33. The assumed flow rate calculated by the standard flow rate calculation unit 47 is transmitted from the standard flow rate calculation unit 47 to the assumed flow rate storage unit 32 of the storage unit 30 and stored therein. Alternatively, the "assumed flow rate" is a flow rate value determined and assumed in advance by the operator or the like, and is an assumed value of the flow rate of blood flowing inside the circulation circuit 1R. The assumed flow rate determined by the operator or the like is input by, for example, the touch panel 52 and is stored in the assumed flow rate storage unit 32 of the storage unit 30.

In addition, the display processing unit 41 executes a process of displaying a standard pressure calculated based on the assumed flow rate by referring to the standard information stored in the standard information storage unit 33 of the storage unit 30 and an actual pressure related to a device, calculated based on the actual pressure measured by the pressure measurement unit 20 as an actually measured value of the pressure of blood flowing inside the circulation circuit 1R and the actual flow rate measured by the flow rate measurement unit 24, on the external monitor 16. Details of the standard information and the standard pressure will be described later.

The notification processing unit 42 executes a process of providing notification of a warning when a predetermined condition is satisfied. For example, the warning notification is executed by displaying warning information (warning content) of the storage unit 30 on the external monitor 16. Alternatively, the warning notification may be executed, for example, by generation of light or sound.

The standard pressure calculation unit 43 refers to the standard information stored in the standard information storage unit 33 and calculates a standard pressure based on the assumed flow rate stored in the assumed flow rate storage unit 32. The standard information is information that represents a relation between the blood flow rate and a pressure loss occurring in each device such as the blood removing catheter 5, the oxygenator 2, and the blood feeding catheter 6 provided in the circulation circuit 1R. The standard pressure refers to the pressure loss occurring in each device provided in the circulation circuit 1R when the blood flow rate is the assumed flow rate.

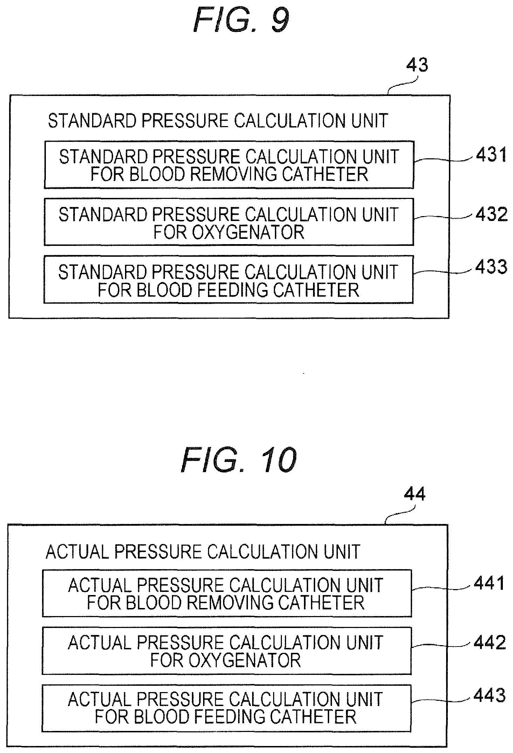

Here, the standard information storage unit 33 and the standard information stored in the standard information storage unit 33 will be further described with reference to Figs. 5 to 8. In addition, the standard pressure calculation unit 43 will be further described with reference to Fig. 9.

Fig. 5 is a schematic diagram illustrating the standard information storage unit of the present embodiment.

Fig. 6 is a graph illustrating an example of standard information related to the blood removing catheter of the present embodiment.

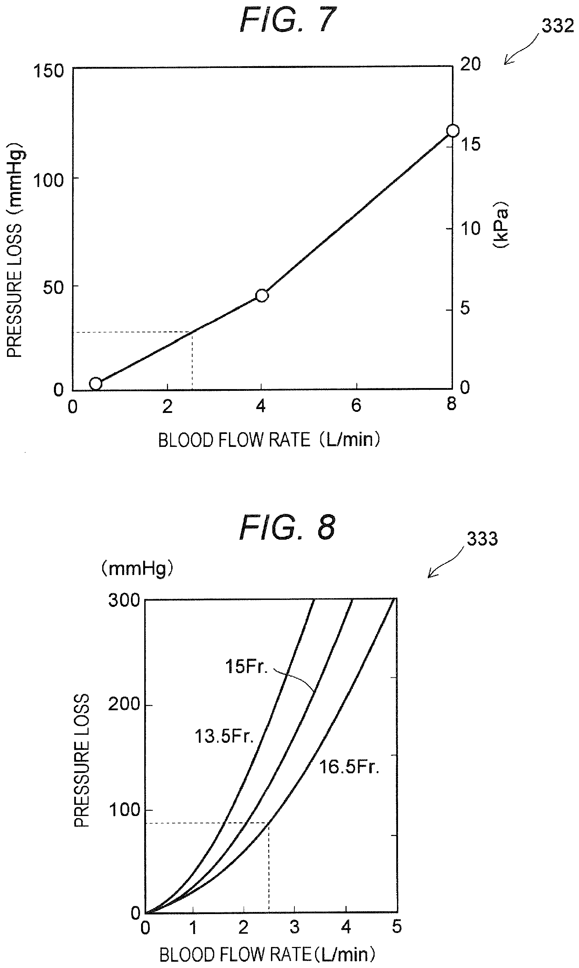

Fig. 7 is a graph illustrating an example of standard information related to the oxygenator of the present embodiment.

Fig. 8 is a graph illustrating an example of standard information related to the blood feeding catheter of the present embodiment.

Fig. 9 is a schematic diagram illustrating the standard pressure calculation unit of the present embodiment.

As illustrated in Fig. 5, the standard information stored in the standard information storage unit 33 includes standard information 331 related to the blood removing catheter 5, standard information 332 related to the oxygenator 2, standard information 333 related to the blood feeding catheter 6, and the standard information 334 related to the circulation circuit 1R.

An example of the standard information 331 related to the blood removing catheter 5 is given as illustrated in the graph illustrated in Fig. 6. That is, the horizontal axis of the graph illustrated in Fig. 6 is a flow rate (L/min) of blood flowing through the blood removing catheter 5. The vertical axis of the graph illustrated in Fig. 6 is a pressure loss (mmHg) that occurs in the blood removing catheter 5. As a condition of the graph illustrated in Fig. 6, the blood is bovine blood. Each of "18 Fr.", "19.5 Fr.", and "21 Fr." described in the graph of Fig. 6 represents a French size of the blood removing catheter 5. For example, when the flow rate of blood flowing through the blood removing catheter 5 is 2.5 L/min in the case where the French size of the blood removing catheter 5 is "21 Fr.", the pressure loss that occurs in the blood removing catheter 5 is about 63 mmHg.

An example of the standard information 332 related to oxygenator 2 is given as illustrated in the graph illustrated in Fig. 7. That is, the horizontal axis of the graph illustrated in Fig. 7 is a flow rate (L/min) of blood flowing through the oxygenator 2. The vertical axis of the graph illustrated in Fig. 7 is a pressure loss (mmHg) that occurs in the oxygenator 2. As a condition of the graph illustrated in Fig. 7, the blood is bovine blood. For example, when the flow rate of blood flowing through the oxygenator 2 is 2. 5L/min, the pressure loss generated in the oxygenator 2 is about 28 mmHg.

An example of the standard information 333 related to the blood feeding catheter 6 is given as illustrated in the graph illustrated in Fig. 8. That is, the horizontal axis of the graph illustrated in Fig. 8 is a flow rate (L/min) of blood flowing through the blood feeding catheter 6. The vertical axis of the graph illustrated in Fig. 8 is a pressure loss (mmHg) that occurs in the blood feeding catheter 6. As a condition of the graph illustrated in Fig. 8, the blood is bovine blood. Each of "13.5 Fr.", "15 Fr." and "16.5 Fr." described in the graph of Fig. 8 represents a French size of the blood feeding catheter 6. For example, when the flow rate of blood flowing through the blood feeding catheter 6 is 2.5 L/min in the case where the French size of the blood feeding catheter 6 is "16.5 Fr.", the pressure loss that occurs in the blood feeding catheter 6 is about 87 mmHg.

The standard information 334 related to the circulation circuit 1R includes the standard information 331 related to the blood removing catheter 5, the standard information 332 related to the oxygenator 2, the standard information 333 related to the blood feeding catheter 6, and standard information related to tubes used in the circulation circuit 1R. Examples of the standard information related to the tubes used in the circulation circuit 1R can include standard information related to the blood removal tube 11, standard information related to the blood feeding tube 12, and the like.

As illustrated in Fig. 9, the standard pressure calculation unit 43 of the present embodiment includes a standard pressure calculation unit 431 related to the blood removing catheter 5, a standard pressure calculation unit 432 related to the oxygenator 2, and a standard pressure calculation unit 433 related to the blood feeding catheter 6. The standard pressure calculation unit 431 related to the blood removing catheter 5 refers to the standard information 331 related to the blood removing catheter 5 and calculates a standard pressure related to the blood removing catheter 5 based on the assumed flow rate stored in the assumed flow rate storage unit 32. The standard pressure related to the blood removing catheter 5 refers to a pressure loss that occurs in the blood removing catheter 5 when the blood flow rate is the assumed flow rate. For example, when the assumed flow rate stored in the assumed flow rate storage unit 32 is 2.5 L/min, the standard pressure calculation unit 431 related to the blood removing catheter 5 calculates about 63 mmHg as the standard pressure related to the blood removing catheter 5.

The standard pressure calculation unit 432 related to the oxygenator 2 refers to the standard information 332 related to the oxygenator 2 and calculates a standard pressure related to the oxygenator 2 based on the assumed flow rate stored in the assumed flow rate storage unit 32. The standard pressure related to the oxygenator 2 refers to a pressure loss that occurs in the oxygenator 2 when the blood flow rate is the assumed flow rate. For example, when the assumed flow rate stored in the assumed flow rate storage unit 32 is 2.5 L/min, the standard pressure calculation unit 432 related to the oxygenator 2 calculates about 28 mmHg as the standard pressure related to the oxygenator 2.

The standard pressure calculation unit 433 related to the blood feeding catheter 6 refers to the standard information 333 related to the blood feeding catheter 6, and calculates a standard pressure related to the blood feeding catheter 6 based on the assumed flow rate stored in the assumed flow rate storage unit 32. The standard pressure related to the blood feeding catheter 6 refers to a pressure loss that occurs in the blood feeding catheter 6 when the blood flow rate is the assumed flow rate. For example, when the assumed flow rate stored in the assumed flow rate storage unit 32 is 2.5 L/min, the standard pressure calculation unit 433 related to the blood feeding catheter 6 calculates about 87 mmHg as the standard pressure related to the blood feeding catheter 6.

Subsequently, the main configuration of the extracorporeal circulation management device 10 will be further described returning to Fig. 4.

The actual pressure calculation unit 44 calculates a pressure loss that actually occurs in each device, such as the blood removing catheter 5, the oxygenator 2, and the blood feeding catheter 6 provided in the circulation circuit 1R, based on the actual pressure measured by the pressure measurement unit 20 and the actual flow rate measured by the flow rate measurement unit 24.

Here, the actual pressure calculation unit 44 will be further described with reference to Fig. 10.

Fig. 10 is a schematic diagram illustrating the actual pressure calculation unit of the present embodiment.

As illustrated in Fig. 10, the actual pressure calculation unit 44 of the present embodiment includes an actual pressure calculation unit 441 related to the blood removing catheter 5, an actual pressure calculation unit 442 related to the oxygenator 2, and an actual pressure calculation unit 443 related to the blood feeding catheter 6.

The actual pressure calculation unit 441 related to the blood removing catheter 5 calculates an actual pressure related to the blood removing catheter 5 based on the actual pressure measured by the first pressure sensor 21 and the actual flow rate measured by the flow rate measurement unit 24. Specifically, the actual pressure calculation unit 441 related to the blood removing catheter 5 calculates a value, obtained by subtracting a blood pressure (for example, central venous pressure (CVP)) displayed on the biological monitor 15 from an actually measured value measured by the first pressure sensor 21, as a pressure loss that actually occurs in the blood removing catheter 5.

Incidentally, in the extracorporeal circulation device 1A according to the modification described above with respect to Fig. 2, the actual pressure calculation unit 441 related to the blood removing catheter 5 calculates the actual pressure related to the blood removing catheter 5 based on the actual pressure measured by the first pressure sensor 21, the head of the centrifugal pump 3, and the actual flow rate measured by the flow rate measurement unit 24. Specifically, the actual pressure calculation unit 441 related to the blood removing catheter 5 calculates a value, obtained by subtracting the head of the centrifugal pump 3 from the actually measured value measured by the first pressure sensor 21, as a pressure value of blood flowing inside the blood removal tube 11 (blood pressure inside the blood removal tube 11). Further, the actual pressure calculation unit 441 related to the blood removing catheter 5 calculates a value, obtained by subtracting a blood pressure displayed on the biological monitor 15 (blood pressure of the patient P detected by the blood pressure measurement unit 25: for example, central venous pressure (CVP)) from the blood pressure in the blood removal tube 11, as a pressure loss that actually occurs in the blood removing catheter 5.

The actual pressure calculation unit 442 related to the oxygenator 2 calculates an actual pressure related to the oxygenator 2 based on the actual pressure measured by the second pressure sensor 22, the actual pressure measured by the third pressure sensor 23, and the actual flow rate measured by the flow rate measurement unit 24. Specifically, the actual pressure calculation unit 442 related to the oxygenator 2 calculates a value, obtained by subtracting an actually measured value measured by the second pressure sensor 22 from an actually measured value measured by the third pressure sensor 23, as a pressure loss that actually occurs in the oxygenator 2.

Incidentally, in the extracorporeal circulation device 1A according to the modification described above with respect to Fig. 2, the actual pressure calculation unit 442 related to the oxygenator 2 calculates the actual pressure related to the oxygenator 2 based on the actual pressure measured by the first pressure sensor 21, the actual pressure measured by the second pressure sensor 22, and the actual flow rate measured by the flow rate measurement unit 24. Specifically, the actual pressure calculation unit 442 related to the oxygenator 2 calculates a value, obtained by subtracting the actually measured value measured by the first pressure sensor 21 from the actually measured value measured by the second pressure sensor 22, as a pressure loss that actually occurs in the oxygenator 2. Alternatively, in the extracorporeal circulation device 1A according to the modification described with respect to Fig. 2, the actual pressure calculation unit 442 related to the oxygenator 2 calculates a value, obtained by adding the head of the centrifugal pump 3 to the actually measured value measured by the first pressure sensor 21, as a pressure value flowing in a tube between the centrifugal pump 3 and the oxygenator 2 (a blood pressure in the tube between the centrifugal pump 3 and the oxygenator 2) when the first pressure sensor 21 is provided in the circulation circuit 1R between the blood removing catheter 5 and the centrifugal pump 3. Further, the actual pressure calculation unit 442 related to the oxygenator 2 calculates a value, obtained by subtracting the blood pressure in the tube between the centrifugal pump 3 and the oxygenator 2 from the actually measured value measured by the second pressure sensor 22, as the pressure loss that actually occurs in the oxygenator 2.

The actual pressure calculation unit 443 related to the blood feeding catheter 6 calculates an actual pressure related to the blood feeding catheter 6 based on the actual pressure measured by the third pressure sensor 23 and the actual flow rate measured by the flow rate measurement unit 24. Specifically, the actual pressure calculation unit 443 related to the blood feeding catheter 6 calculates a value, obtained by subtracting the actually measured value measured by the third pressure sensor 23 from a blood pressure (for example, average blood pressure) displayed on the biological monitor 15, as a pressure loss that actually occurs in the blood feeding catheter 6.

Incidentally, in the extracorporeal circulation device 1A according to the modification described with respect to Fig. 2, the actual pressure calculation unit 443 related to the blood feeding catheter 6 calculates the actual pressure related to the blood feeding catheter 6 based on the actual pressure measured by the second pressure sensor 22 and the actual flow rate measured by the flow rate measurement unit 24. Specifically, the actual pressure calculation unit 443 related to the blood feeding catheter 6 calculates a value, obtained by subtracting the actually measured value measured by the second pressure sensor 22 from the blood pressure (for example, average blood pressure) displayed on the biological monitor 15, as the pressure loss that actually occurs in the blood feeding catheter 6.

Subsequently, the main configuration of the extracorporeal circulation management device 10 will be further described returning to Fig. 4.

The differential flow rate calculation unit 45 calculates a differential flow rate representing a difference between an assumed flow rate and an actual flow rate. Specifically, the differential flow rate calculation unit 45 calculates a difference between the assumed flow rate stored in the assumed flow rate storage unit 32 and the actually measured value (actual flow rate) measured by the flow rate measurement unit 24 as the differential flow rate.

The differential pressure calculation unit 46 calculates a differential pressure representing a difference between a standard pressure and an actual pressure. Specifically, the differential pressure calculation unit 46 calculates a difference between the standard pressure calculated by the standard pressure calculation unit 43 and the actual pressure related to each device calculated by the actual pressure calculation unit 44 as the differential pressure.

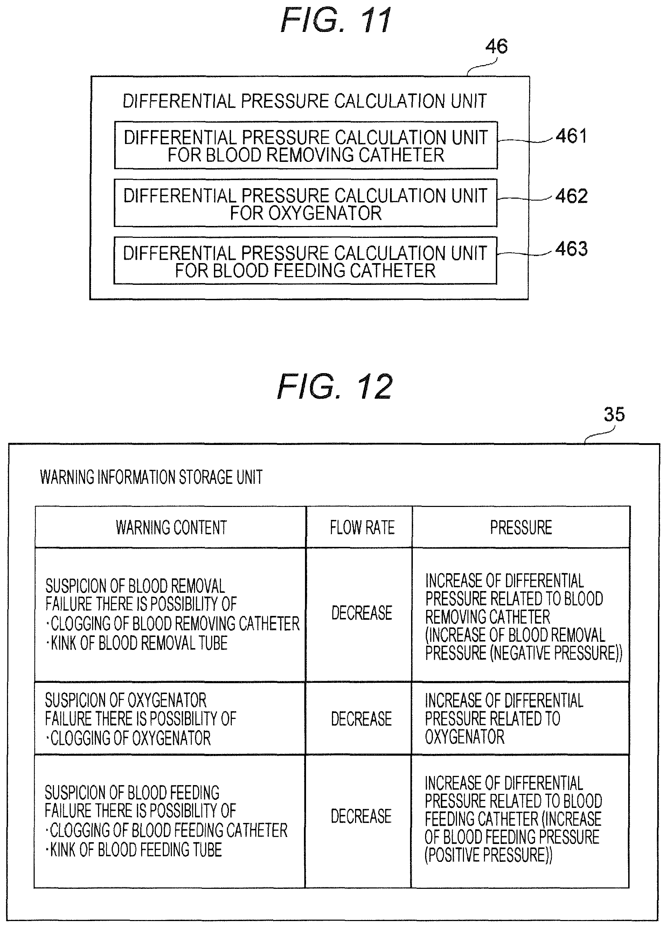

Here, the differential pressure calculation unit 46 will be further described with reference to Fig. 11.

Fig. 11 is a schematic diagram illustrating the differential pressure calculation unit of the present embodiment.

As illustrated in Fig. 11, the differential pressure calculation unit 46 of the present embodiment includes a differential pressure calculation unit 461 related to the blood removing catheter 5, a differential pressure calculation unit 462 related to the oxygenator 2, and a differential pressure calculation unit 463 related to the blood feeding catheter 6.

The differential pressure calculation unit 461 related to the blood removing catheter 5 calculates a differential pressure related to the blood removing catheter 5 which represents a difference between the standard pressure related to the blood removing catheter 5 and the actual pressure related to the blood removing catheter 5. Specifically, the differential pressure calculation unit 461 related to the blood removing catheter 5 calculates a difference between the standard pressure related to the blood removing catheter 5 calculated by the standard pressure calculation unit 431 related to the blood removing catheter 5 and the actual pressure related to the blood removing catheter 5 calculated by the actual pressure calculation unit 441 related to the blood removing catheter 5 as the differential pressure related to the blood removing catheter 5.

The differential pressure calculation unit 462 related to the oxygenator 2 calculates a differential pressure related to the oxygenator 2 which represents a difference between the standard pressure related to the oxygenator 2 and the actual pressure related to the oxygenator 2. Specifically, the differential pressure calculation unit 462 related to the oxygenator 2 calculates a difference between the standard pressure related to the oxygenator 2 calculated by the standard pressure calculation unit 432 related to the oxygenator 2 and the actual pressure related to the oxygenator 2 calculated by the actual pressure calculation unit 442 related to the oxygenator 2 as the differential pressure related to the oxygenator 2.

The differential pressure calculation unit 463 related to the blood feeding catheter 6 calculates a differential pressure related to the blood feeding catheter 6 which represents a difference between the standard pressure related to the blood feeding catheter 6 and the actual pressure related to the blood feeding catheter 6. Specifically, the differential pressure calculation unit 463 related to the blood feeding catheter 6 calculates a difference between the standard pressure related to the blood feeding catheter 6 calculated by the standard pressure calculation unit 433 related to the blood feeding catheter 6 and the actual pressure related to the blood feeding catheter 6 calculated by the actual pressure calculation unit 443 related to the blood feeding catheter 6 as the differential pressure related to the blood feeding catheter 6.

Subsequently, the main configuration of the extracorporeal circulation management device 10 will be further described returning to Fig. 4.

The assumed flow rate storage unit 32 stores the assumed flow rate calculated by the standard flow rate calculation unit 47 and transmitted from the standard flow rate calculation unit 47. Alternatively, the assumed flow rate storage unit 32 stores the assumed flow rate determined by the operator or the like and input by, for example, the touch panel 52. The standard information storage unit 33 is given as described above with respect to Figs. 5 to 8.

The pump characteristic storage unit stores information on a characteristic of the centrifugal pump 3. Examples of the information on the characteristic of the centrifugal pump 3 include a graph illustrating a relation between a flow rate (L/min) of blood sent by the centrifugal pump 3 and a head (mmHg) of the centrifugal pump 3. For example, in the graph illustrating the characteristic of the centrifugal pump 3, the relation between the flow rate (L/min) of blood sent by the centrifugal pump 3 and the head (mmHg) of the centrifugal pump 3 is set depending on a rotational speed (rpm) of the centrifugal pump 3.

The warning information storage unit 35 stores warning information (warning content) notification of which is provided by the notification processing unit 42.

Here, the differential pressure calculation unit 46 will be further described with reference to Fig. 12.

Fig. 12 is a schematic diagram illustrating the warning information storage unit of the present embodiment.

As illustrated in Fig. 12, the warning information storage unit 35 stores warning information data that can be assumed by combining a flow rate and a pressure. For example, in a case where the actual flow rate measured by the flow rate measurement unit 24 decreases and the differential pressure related to the blood removing catheter 5 increases, the notification processing unit 42 refers to the warning information stored in the warning information storage unit 35 and displays that there is a suspicion of blood removal failure on the external monitor 16. For example, the notification processing unit 42 displays that there is a "possibility of clogging of the blood removing catheter 5" due to the distal end of the blood removing catheter 5 coming into contact with a blood vessel wall or there is a "possibility of a kink of the blood removal tube 11" on the external monitor 16 as the suspicion of the blood removal failure. Incidentally, the case where the differential pressure related to the blood removing catheter 5 increases corresponds to, for example, a case where a blood removal pressure (negative pressure) increases.

In addition, for example, in a case where the actual flow rate measured by the flow rate measurement unit 24 decreases and the differential pressure related to the oxygenator 2 increases, the notification processing unit 42 refers to the warning information stored in the warning information storage unit 35 and displays that there is a suspicion of oxygenator failure on the external monitor 16. For example, the notification processing unit 42 displays that there is a "possibility of clogging of the oxygenator 2" due to the life of the oxygenator 2 or the like on the external monitor 16 as the suspicion of oxygenator failure.

In addition, for example, in a case where the actual flow rate measured by the flow rate measurement unit 24 decreases and the differential pressure related to the blood feeding catheter 6 increases, the notification processing unit 42 refers to the warning information stored in the warning information storage unit 35 and displays that there is a suspicion of blood feeding failure on the external monitor 16. For example, the notification processing unit 42 displays that there is a "possibility of clogging of the blood feeding catheter 6" due to a distal end of the blood feeding catheter 6 coming into contact with a blood vessel wall or there is a "possibility of a kink of the blood feeding tube 12" on the external monitor 16 as the suspicion of the blood feeding failure. Incidentally, the case where the differential pressure of the blood feeding catheter 6 increases corresponds to, for example, a case where a blood feeding pressure (positive pressure) increases.

Subsequently, the main configuration of the extracorporeal circulation management device 10 will be further described returning to Fig. 4.

The communication unit 53 communicates with the drive motor 4, the biological monitor 15, the external monitor 16, the pressure measurement unit 20, and the flow rate measurement unit 24, and transmits and receives various types of information and various signals.

Next, an example of an image displayed on the external monitor 16 of the present embodiment will be described with reference to the drawings.

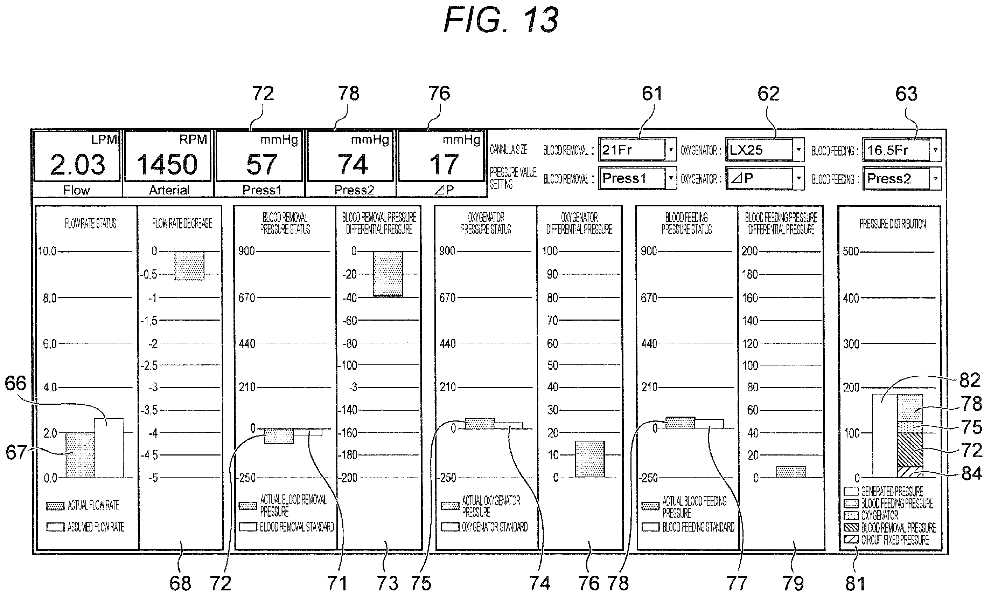

Fig. 13 is a schematic diagram illustrating an example of the image displayed on the external monitor according to the present embodiment.

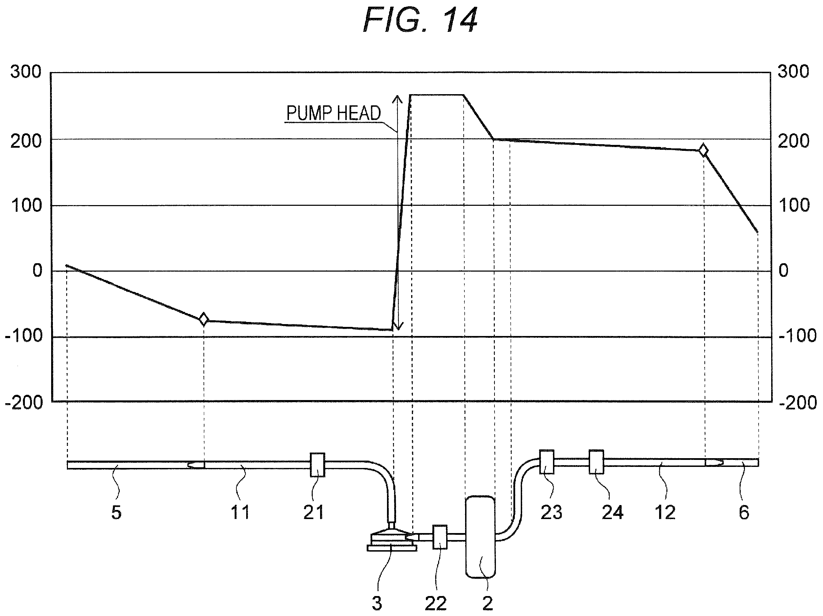

Fig. 14 is a schematic diagram illustrating an example of a relation between arrangements of the circulation circuit and each device of the extracorporeal circulation device according to the present embodiment and a pressure loss.

As illustrated in Fig. 13, the display processing unit 41 of the extracorporeal circulation management device 10 according to the present embodiment displays an assumed flow rate 66 stored in the assumed flow rate storage unit 32 and an actual flow rate 67 measured by the flow rate measurement unit 24 on the external monitor 16. In addition, the display processing unit 41 displays standard pressures 71, 74, and 77 calculated by the standard pressure calculation unit 43 and actual pressures 72, 75, and 78 related to the respective devices calculated by the actual pressure calculation unit 44 on the external monitor 16.

Further, the display processing unit 41 displays a differential flow rate 68, which represents a difference between the assumed flow rate 66 and the actual flow rate 67, and differential pressures 73, 76, and 79 each of which represents a difference between each of the standard pressures 71, 74, and 77 and each of the actual pressures 72, 75, and 78 related to the respective devices on the external monitor 16.

To be specific, the display processing unit 41 displays the standard pressure 71 related to the blood removing catheter 5, calculated based on the assumed flow rate 66 by the standard pressure calculation unit 431 related to the blood removing catheter 5, and the actual pressure 72 related to the blood removing catheter 5, calculated by the actual pressure calculation unit 441 related to the blood removing catheter 5 based on the actual pressure measured by the first pressure sensor 21 and the actual flow rate 67 measured by the flow rate measurement unit 24, on the external monitor 16. In the example of the image illustrated in Fig. 13, the display processing unit 41 displays the standard pressure 71 related to the blood removing catheter 5 and the actual pressure 72 related to the blood removing catheter 5 in parallel. Further, the display processing unit 41 displays the differential pressure 73 related to the blood removing catheter 5 calculated by the differential pressure calculation unit 461 related to the blood removing catheter 5, which represents the difference between the standard pressure 71 related to the blood removing catheter 5 and the actual pressure 72 related to the blood removing catheter 5, on the external monitor 16. In the example of the image illustrated in Fig. 13, the display processing unit 41 displays the standard pressure 71 related to the blood removing catheter 5, the actual pressure 72 related to the blood removing catheter 5, and the differential pressure 73 related to the blood removing catheter 5 in parallel.

In addition, the display processing unit 41 displays the standard pressure 74 related to the oxygenator 2, calculated based on the assumed flow rate 66 by the standard pressure calculation unit 432 related to the oxygenator 2, and the actual pressure 75 related to the oxygenator 2, calculated by the actual pressure calculation unit 442 related to the oxygenator 2 based on the actual pressures measured by the second pressure sensor 22 and the third pressure sensor 23 and the actual flow rate 67 measured by the flow rate measurement unit 24, on the external monitor 16. In the example of the image illustrated in Fig. 13, the display processing unit 41 displays the standard pressure 74 related to the oxygenator 2 and the actual pressure 75 related to the oxygenator 2 in parallel. Further, the display processing unit 41 displays the differential pressure 76 related to the oxygenator 2 calculated by the differential pressure calculation unit 462 related to the oxygenator 2, which represents the difference between the standard pressure 74 related to the oxygenator 2 and the actual pressure 75 related to the oxygenator 2, on the external monitor 16. In the example of the image illustrated in Fig. 13, the display processing unit 41 displays the standard pressure 74 related to the oxygenator 2, the actual pressure 75 related to the oxygenator 2, and the differential pressure 76 related to the oxygenator 2 in parallel.

In addition, the display processing unit 41 displays the standard pressure 77 related to the blood feeding catheter 6, calculated based on the assumed flow rate 66 by the standard pressure calculation unit 433 related to the blood feeding catheter 6, and the actual pressure 78 related to the blood feeding catheter 6, calculated by the actual pressure calculation unit 443 related to the blood feeding catheter 6 based on the actual pressure measured by the third pressure sensor 23 and the actual flow rate 67 measured by the flow rate measurement unit 24, on the external monitor 16. In the example of the image illustrated in Fig. 13, the display processing unit 41 displays the standard pressure 77 related to the blood feeding catheter 6 and the actual pressure 78 related to the blood feeding catheter 6 in parallel. Further, the display processing unit 41 displays the differential pressure 79 related to the blood feeding catheter 6 calculated by the differential pressure calculation unit 463 related to the blood feeding catheter 6, which represents the difference between the standard pressure 77 related to the blood feeding catheter 6 and the actual pressure 78 related to the blood feeding catheter 6, on the external monitor 16. In the example of the image illustrated in Fig. 13, the display processing unit 41 displays the standard pressure 77 related to the blood feeding catheter 6, the actual pressure 78 related to the blood feeding catheter 6, and the differential pressure 79 related to the blood feeding catheter 6 in parallel.

According to the extracorporeal circulation device 1 and the extracorporeal circulation management device 10 of the present embodiment, the operator or the like can easily grasp a discrepancy or deviation between the assumed flow rate 66 of blood in the circulation circuit 1R and the actual flow rate 67 of the blood actually flowing in the circulation circuit 1R by confirming the external monitor 16. In addition, the operator or the like can easily grasp a discrepancy or deviation between each of the standard pressures 71, 74, and 77 calculated based on the assumed flow rate 66 and each of the actual pressures 72, 75, and 78 related to the respective devices (the blood removing catheter 5, the oxygenator 2, and the blood feeding catheter 6) by confirming the external monitor 16. Therefore, when there is the discrepancy or deviation between the assumed flow rate 66 and the actual flow rate 67, the operator or the like can easily grasp the occurrence of the discrepancy or deviation between the assumed flow rate 66 and the actual flow rate 67 and grasp a location of the circulation circuit 1R where the discrepancy or deviation between the standard pressure 71, 74, or 77 and the actual pressure 72, 75, or 78 has occurred and easily grasp that a cause of circulation failure exists near the location by confirming the external monitor 16. That is, the location of the circulation circuit 1R where the discrepancy or deviation between the standard pressure 71, 74, or 77 and the actual pressure 72, 75, or 78 has occurred corresponds to a portion where the pressure measurement unit 20 is provided or a portion of each device provided near the pressure measurement unit 20. In this manner, the operator or the like can easily grasp the cause of the circulation failure in the extracorporeal circulation.

In addition, the differential flow rate 68 and the differential pressures 73, 76, and 79 are further displayed on the external monitor 16 as described above. As a result, the operator or the like can more easily grasp the discrepancy or deviation between the assumed flow rate 66 and the actual flow rate 67 and the discrepancy or deviation between the standard pressure 71, 74, or 77 and the actual pressure 72, 75, or 78 by confirming the external monitor 16. As described above, the standard pressure 71, 74, or 77 is calculated based on the standard information 331, 332, or 333 stored in the standard information storage unit 33 and the assumed flow rate 66 stored in the assumed flow rate storage unit 32. That is, the standard pressures 71, 74, and 77 change depending on the assumed flow rate 66. Therefore, a discrepancy or deviation sometimes occurs between the standard pressure 71, 74, or 77 and the actual pressure 72, 75, or 78 even if the actual pressure 72, 75, or 78 does not change at first glance. On the other hand, according to the extracorporeal circulation device 1 and the extracorporeal circulation management device 10 of the present embodiment, the operator or the like can more easily grasp the discrepancy or deviation between the standard pressure 71, 74, or 77 and the actual pressure 72, 75, or 78 by confirming the external monitor 16.

In addition, as illustrated in Fig. 13, the standard pressure 71, the actual pressure 72, and the differential pressure 73 related to the blood removing catheter 5, the standard pressure 74, the actual pressure 75, and the differential pressure 76 related to the oxygenator 2, and the standard pressure 77, the actual pressure 78, and the differential pressure 79 related to the blood feeding catheter 6 are displayed in parallel according to the actual arrangement of each device or per actual arrangement of each device. As a result, the operator or the like can more easily grasp the discrepancy or deviation between the standard pressure 71, 74, or 77 and the actual pressure 72, 75, or 78, and more concretely and easily grasp a location of the circulation circuit 1R where the discrepancy or deviation between the standard pressure 71, 74, or 77 and the actual pressure 72, 75, or 78 has occurred and easily grasp that a cause of circulation failure exists near the location by confirming the external monitor 16 as one display unit.

In addition, as illustrated in Fig. 13, all the assumed flow rate 66, the actual flow rate 67, the differential flow rate 68, the standard pressure 71 related to the blood removing catheter 5, the standard pressure 74 related to the oxygenator 2, the standard pressure 77 related to the blood feeding catheter 6, the actual pressure 72 related to the blood removing catheter 5, the actual pressure 75 related to the oxygenator 2, the actual pressure 78 related to the blood feeding catheter 6, the differential pressure 73 related to the blood removing catheter 5, the differential pressure 76 related to the oxygenator 2, and the differential pressure 79 related to the blood feeding catheter 6 are displayed simultaneously and in parallel on the external monitor 16 as one display unit. As a result, the operator or the like can more easily grasp the discrepancy or deviation between the standard pressure 71, 74, or 77 and the actual pressure 72, 75, or 78, and more concretely and easily grasp a location of the circulation circuit 1R where the discrepancy or deviation between the standard pressure 71, 74, or 77 and the actual pressure 72, 75, or 78 has occurred and easily grasp that a cause of circulation failure exists near the location by confirming the external monitor 16 as one display unit. That is, a location of the circulation circuit 1R where a discrepancy or deviation between the standard pressure 71, 74, or 77 and the actual pressure 72, 75, or 78 has occurred corresponds to a portion where a pressure sensor detecting an abnormal value among the first pressure sensor 21, the second pressure sensor 22, and the third pressure sensor 23 is provided or a portion of an instrument element provided near the pressure sensor detecting the abnormal value among the blood removing catheter 5, the oxygenator 2, and the blood feeding catheter 6. In addition, the image (bar graph) displayed on the external monitor 16 changes in real time. As a result, the operator or the like can immediately grasp the discrepancy or deviation between the standard pressure 71, 74, or 77 and the actual pressure 72, 75, or 78.

In addition, as illustrated in Fig. 13, the display processing unit 41 displays a French size 61 of the blood removing catheter 5, a type 62 of the oxygenator 2, and a French size 63 of the blood feeding catheter 6 on the external monitor 16. The operator or the like can easily change settings of at least any of the French size 61 of the blood removing catheter 5, the type 62 of the oxygenator 2, and the French size 63 of the blood feeding catheter 6 on the image displayed on the external monitor 16. As a result, the operator or the like can change settings of the standard pressure 71 related to the blood removing catheter 5, the standard pressure 74 related to the oxygenator 2, and the standard pressure 77 related to the blood feeding catheter 6 by changing settings of at least any of the French size 61 of the blood removing catheter 5, the type 62 of the oxygenator 2, and the French size 63 of the blood feeding catheter 6 on the image displayed on the external monitor 16. A relation between the French size 61 of the blood removing catheter 5 and the standard pressure 71 related to the blood removing catheter 5 and a relation between the French size 63 of the blood feeding catheter 6 and the standard pressure 77 related to the blood feeding catheter 6 are the same as those described above with respect to Figs. 6 and 8.

Further, the display processing unit 41 displays an overall pressure distribution 81 of the extracorporeal circulation device 1 on the external monitor 16. The overall pressure distribution 81 of the extracorporeal circulation device 1 includes a head 82 of the centrifugal pump 3, the actual pressure 72 related to the blood removing catheter 5, the actual pressure 75 related to the oxygenator 2, the actual pressure 78 related to the blood feeding catheter 6, and a circuit fixed pressure 84. The circuit fixed pressure 84 is a pressure applied to a tube connecting the blood removing catheter 5, the centrifugal pump 3, the oxygenator 2, and the blood feeding catheter 6, and is, for example, a pressure applied to the blood removal tube 11, a pressure applied to the blood feeding tube 12, or the like.

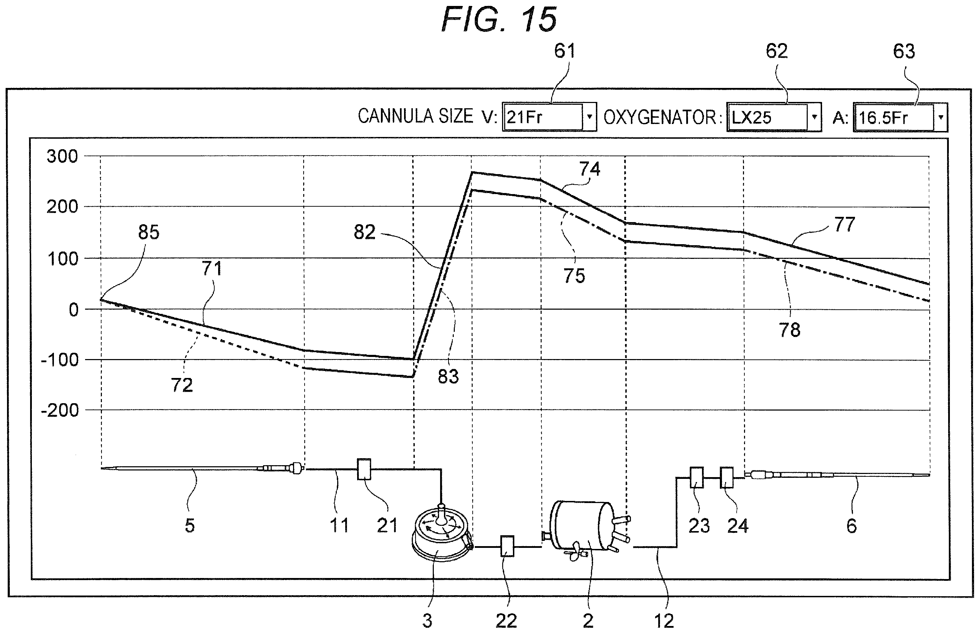

Fig. 15 is a schematic diagram illustrating another example of the image displayed on the external monitor of the present embodiment.

On the image displayed on the external monitor 16 described above with respect to Fig. 13, the standard pressure 71 related to the blood removing catheter 5, the standard pressure 74 related to the oxygenator 2, the standard pressure 77 related to the blood feeding catheter 6, the actual pressure 72 related to the blood removing catheter 5, the actual pressure 75 related to the oxygenator 2, the actual pressure 78 related to the blood feeding catheter 6, the differential pressure 73 related to the blood removing catheter 5, the differential pressure 76 related to the oxygenator 2, the differential pressure 79 related to the blood feeding catheter 6, and the head 82 of the centrifugal pump 3 are illustrated using bar graphs. In contrast, on the image illustrated in Fig. 15, blood pressure information displayed on the biological monitor 15, such as the standard pressure 71 related to the blood removing catheter 5, the standard pressure 74 related to the oxygenator 2, the standard pressure 77 related to the blood feeding catheter 6, the actual pressure 72 related to the blood removing catheter 5, the actual pressure 75 related to the oxygenator 2, the actual pressure 78 related to the blood feeding catheter 6, the differential pressure 73 related to the blood removing catheter 5, the differential pressure 76 related to the oxygenator 2, and the differential pressure 79 related to the blood feeding catheter 6, the standard head 82 of the centrifugal pump 3, an actual head 83 of the centrifugal pump 3, and a central venous pressure (CVP) 85, is illustrated using a line graph. In this regard, the image illustrated in Fig. 15 is different from the image illustrated in Fig. 13.

On the image illustrated in Fig. 15, the operator or the like can more easily grasp a discrepancy or deviation between the standard pressure 71, 74, or 77 and the actual pressure 72, 75, or 78 while confirming the relation between the arrangements of the circulation circuit 1R and the respective devices of the extracorporeal circulation device 1 and the pressure loss using the external monitor 16. In other words, the operator or the like can more concretely and easily grasp a location of the circulation circuit 1R where the discrepancy or deviation between the standard pressure 71, 74, or 77 and the actual pressure 72, 75, or 78 has occurred, and easily grasp that a cause of circulation failure exists near the location. In addition, the image (line graph) displayed on the external monitor 16 changes in real time. As a result, the operator or the like can immediately grasp the discrepancy or deviation between the standard pressure 71, 74, or 77 and the actual pressure 72, 75, or 78.

In the example of the image illustrated in Fig. 15, it can be seen that the discrepancy or deviation occurs between the standard pressure 71 related to the blood removing catheter 5 and the actual pressure 72 related to the blood removing catheter 5. On the other hand, it can be seen that there is no discrepancy or deviation between the standard pressure 74 related to the oxygenator 2 and the actual pressure 75 related to the oxygenator 2, between the standard pressure 77 related to the blood feeding catheter 6 and the actual pressure 78 related to the blood feeding catheter 6, and between the standard head 82 of the centrifugal pump 3 and the actual head 83 of the centrifugal pump 3. As a result, in the example of the image illustrated in Fig. 15, the operator or the like can grasp that there is a "possibility of clogging of the blood removing catheter 5" due to the distal end of the blood removing catheter 5 coming into contact with the blood vessel wall or there is a "possibility of a kink of the blood removal tube 11" as a suspicion of blood removal failure.

Next, a description will be given with reference to the drawings regarding control executed as the computer 51 of the extracorporeal circulation management device 10 according to the present embodiment reads the program 31 (extracorporeal circulation management program) stored in the storage unit 30.

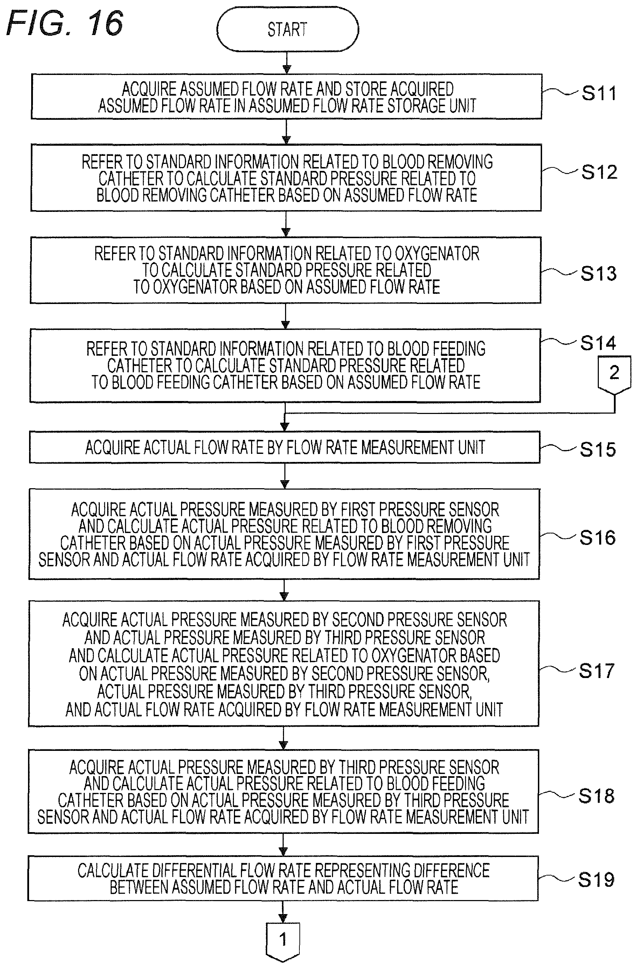

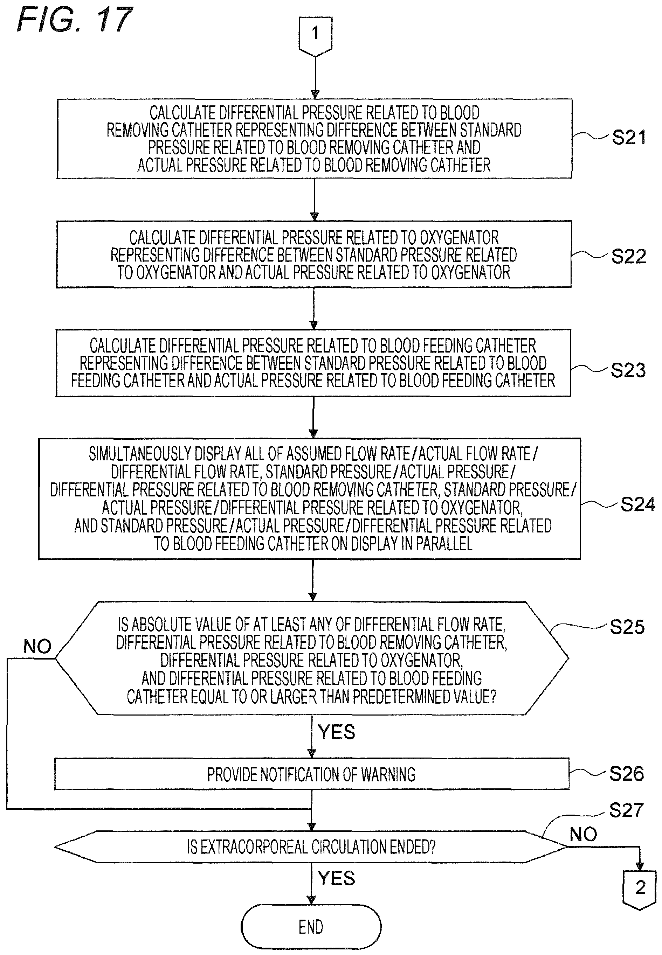

Figs. 16 and 17 are flowcharts illustrating examples of the control executed by the computer of the extracorporeal circulation management device according to the present embodiment.

First, in step S11, the control unit 40 stores, for example, the assumed flow rate 66, input by the operator or the like in response to the operator's operation or the like on the touch panel 52, in the assumed flow rate storage unit 32. Alternatively, the control unit 40 stores the assumed flow rate 66, calculated by the standard flow rate calculation unit 47 using patient information (for example, height, weight, and the like) input by the touch panel 52 in response to the operator's operation on the touch panel 52 and the standard information 334 related to the circulation circuit 1R stored in the standard information storage unit 33, in the assumed flow rate storage unit 32. Subsequently, in step S12, the standard pressure calculation unit 431 related to the blood removing catheter 5 refers to the standard information 331 related to the blood removing catheter 5 stored in the standard information storage unit 33 and calculates the standard pressure 71 related to the blood removing catheter 5 based on the assumed flow rate 66 stored in the assumed flow rate storage unit 32.

Subsequently, in step S13, the standard pressure calculation unit 432 related to the oxygenator 2 refers to the standard information 332 related to the oxygenator 2 stored in the standard information storage unit 33 and calculates the standard pressure 74 related to the oxygenator 2 based on the assumed flow rate 66 stored in the assumed flow rate storage unit 32. Subsequently, in step S14, the standard pressure calculation unit 433 related to the blood feeding catheter 6 refers to the standard information 333 related to the blood feeding catheter 6 stored in the standard information storage unit 33 and calculates the standard pressure 77 related to the blood feeding catheter 6 based on the assumed flow rate 66 stored in the assumed flow rate storage unit 32.

Incidentally, the order of the processes described above with respect to steps S12 to S14 is not particularly limited. For example, the processes described above with respect to steps S12 to S14 may be executed at the same time. Alternatively, the processes described above with respect to steps S14, S13, and S12 may be executed in this order.

Subsequently, in step S15, the control unit 40 acquires the actual flow rate 67 from the flow rate measurement unit 24 through the communication unit 53. Then, in step S16, the actual pressure calculation unit 441 related to the blood removing catheter 5 acquires an actual pressure (actually measured value) from the first pressure sensor 21 through the communication unit 53, and calculates the actual pressure 72 related to the blood removing catheter 5 based on the actual pressure measured by the first pressure sensor 21 and the actual flow rate 67 acquired from the flow rate measurement unit 24. Specifically, the actual pressure calculation unit 441 related to the blood removing catheter 5 calculates a value, obtained by subtracting a blood pressure (for example, central venous pressure (CVP)) displayed on the biological monitor 15 from an actually measured value measured by the first pressure sensor 21, as a pressure loss that actually occurs in the blood removing catheter 5.

Then, in step S17, the actual pressure calculation unit 442 related to the oxygenator 2 acquires an actual pressure (actually measured value) from each of the second pressure sensor 22 and the third pressure sensor 23 through the communication unit 53, and calculates the actual pressure 75 related to the oxygenator 2 based on the actual pressure measured by the second pressure sensor 22, the actual pressure measured by the third pressure sensor 23, and the actual flow rate 67 acquired from the flow rate measurement unit 24. Specifically, the actual pressure calculation unit 442 related to the oxygenator 2 calculates a value, obtained by subtracting an actually measured value measured by the second pressure sensor 22 from an actually measured value measured by the third pressure sensor 23, as a pressure loss that actually occurs in the oxygenator 2.

Subsequently, in step S18, the actual pressure calculation unit 443 related to the blood feeding catheter 6 acquires the actual pressure (actually measured value) from the third pressure sensor 23 through the communication unit 53, and calculates the actual pressure 78 related to the blood feeding catheter 6 based on the actual pressure measured by the third pressure sensor 23 and the actual flow rate 67 acquired from the flow rate measurement unit 24. Specifically, the actual pressure calculation unit 443 related to the blood feeding catheter 6 calculates a value, obtained by subtracting the actually measured value measured by the third pressure sensor 23 from a blood pressure (for example, average blood pressure) displayed on the biological monitor 15, as a pressure loss that actually occurs in the blood feeding catheter 6.

Subsequently, in step S19, the differential flow rate calculation unit 45 calculates the differential flow rate 68 representing the difference between the assumed flow rate 66 stored in the assumed flow rate storage unit 32 and the actual flow rate 67 acquired from the flow rate measurement unit 24. Subsequently, in step S21, the differential pressure calculation unit 461 related to the blood removing catheter 5 calculates the differential pressure 73 related to the blood removing catheter 5 representing the difference between the standard pressure 71 related to the blood removing catheter 5 calculated by the process described above with respect to step S12 and the actual pressure 72 related to the blood removing catheter 5 calculated by the process described above with respect to step S16.

Subsequently, in step S22, the differential pressure calculation unit 462 related to the oxygenator 2 calculates the differential pressure 76 related to the oxygenator 2 representing the difference between the standard pressure 74 related to the oxygenator 2 calculated by the process described above with respect to step S13 and the actual pressure 75 related to the oxygenator 2 calculated by the process described above with respect to step S17. Subsequently, in step S23, the differential pressure calculation unit 463 related to the blood feeding catheter 6 calculates the differential pressure 79 related to the blood feeding catheter 6 representing the difference between the standard pressure 77 related to the blood feeding catheter 6 calculated by the process described above with respect to step S14 and the actual pressure 78 related to the blood feeding catheter 6 calculated by the process described above with respect to step S18.

Subsequently, in step S24, the display processing unit 41 displays all the assumed flow rate 66, the actual flow rate 67, the differential flow rate 68, the standard pressure 71 related to the blood removing catheter 5, the actual pressure 72 related to the blood removing catheter 5, the differential pressure 73 related to the blood removing catheter 5, the standard pressure 74 related to the oxygenator 2, the actual pressure 75 related to the oxygenator 2, the differential pressure 76 related to the oxygenator 2, the standard pressure 77 related to the blood feeding catheter 6, the actual pressure 78 related to the blood feeding catheter 6, and the differential pressure 79 related to the blood feeding catheter 6 simultaneously and in parallel on the external monitor 16 as one display unit.

Subsequently, in step S25, the control unit 40 determines whether or not an absolute value of at least any of the differential flow rate 68, the differential pressure 73 related to the blood removing catheter 5, the differential pressure 76 related to the oxygenator 2, and the differential pressure 79 related to the blood feeding catheter 6 is equal to or larger than a predetermined value. When the absolute value of at least any of the differential flow rate 68, the differential pressure 73 related to the blood removing catheter 5, the differential pressure 76 related to the oxygenator 2, and the differential pressure 79 related to the blood feeding catheter 6 is equal to or larger than the predetermined value (step S25: YES), the notification processing unit 42 refers to the warning information stored in the warning information storage unit 35, and notifies the external monitor 16 of the warning information. As a result, the operator or the like can more easily grasp occurrence of a discrepancy or deviation between the assumed flow rate 66 and the actual flow rate 67, and further grasp a location of the circulation circuit 1R where the discrepancy or deviation between the standard pressure 71, 74, or 77 and the actual pressure 72, 75, or 78 has occurred and more easily grasp that a cause of circulation failure exists near the location. Incidentally, an example of the warning information notification of which is provided by the notification processing unit 42 is the same as that described above with respect to Fig. 12.

On the other hand, when the absolute value of at least any of the differential flow rate 68, the differential pressure 73 related to the blood removing catheter 5, the differential pressure 76 related to the oxygenator 2, and the differential pressure 79 related to the blood feeding catheter 6 is not equal to or larger than the predetermined value (step S25: NO), the control unit 40 determines in step S27 whether or not the extracorporeal circulation is completed. In addition, in step S27 following step S26, the control unit 40 determines in step S27 whether or not the extracorporeal circulation is completed.

When the extracorporeal circulation is not completed (step S27: NO), the processing returns to step S15, and the control unit 40 acquires the actual flow rate 67 from the flow rate measurement unit 24 through the communication unit 53. On the other hand, when the extracorporeal circulation is completed (step S27: YES), the control unit 40 ends the execution of the program 31 related to the extracorporeal circulation.

According to the program 31 (extracorporeal circulation management program) of the present embodiment, the operator or the like can easily grasp a discrepancy or deviation between the assumed flow rate 66 of blood in the circulation circuit 1R and the actual flow rate 67 of the blood actually flowing in the circulation circuit 1R by confirming the external monitor 16. In addition, the operator or the like can easily grasp a discrepancy or deviation between each of the standard pressures 71, 74, and 77 calculated based on the assumed flow rate 66 and each of the actual pressures 72, 75, and 78 related to the respective devices (the blood removing catheter 5, the oxygenator 2, and the blood feeding catheter 6) by confirming the external monitor 16. Therefore, when there is the discrepancy or deviation between the assumed flow rate 66 and the actual flow rate 67, the operator or the like can easily grasp the occurrence of the discrepancy or deviation between the assumed flow rate 66 and the actual flow rate 67 and grasp a location of the circulation circuit 1R where the discrepancy or deviation between the standard pressure 71, 74, or 77 and the actual pressure 72, 75, or 78 has occurred and easily grasp that a cause of circulation failure exists near the location by confirming the external monitor 16. That is, the location of the circulation circuit 1R where the discrepancy or deviation between the standard pressure 71, 74, or 77 and the actual pressure 72, 75, or 78 has occurred corresponds to a portion where the pressure measurement unit 20 is provided or a portion of each device provided near the pressure measurement unit 20. In this manner, the operator or the like can easily grasp the cause of the circulation failure in the extracorporeal circulation. In addition, the same effects as those described above can be obtained with respect to Figs. 13 and 14.

The embodiments of the present invention have been described above. However, the present invention is not limited to the above embodiments, and various modifications can be made within a range not departing from the scope of claims.

### Reference Signs List

1, 1A Extracorporeal circulation device
1R Circulation circuit
2 Oxygenator
3 Centrifugal pump
4 Drive motor
5 Blood removing catheter
6 Blood feeding catheter
8, 9 Connector
10 Extracorporeal circulation management device
11 Blood removal tube
12 Blood feeding tube
14 Oxygen supply tube
15 Biological monitor
16 External monitor
20 Pressure measurement unit
21 First pressure sensor
22 Second pressure sensor
23 Third pressure sensor
24 Flow rate measurement unit
25 Blood pressure measurement unit
30 Storage unit
31 Program
32 Assumed flow rate storage unit
33 Standard information storage unit
34 Pump characteristic storage unit
35 Warning information storage unit
40 Control unit
41 Display processing unit
42 Notification processing unit
43 Standard pressure calculation unit
44 Actual pressure calculation unit
45 Differential flow rate calculation unit
46 Differential pressure calculation unit
47 Standard flow rate calculation unit
51 Computer
52 Touch panel
53 Communication unit
61 French size of blood removing catheter
62 Type of centrifugal pump
63 French size of blood feeding catheter
66 Assumed flow rate
67 Actual flow rate
68 Differential flow rate
71 Standard pressure related to blood removing catheter
72 Actual pressure related to blood removing catheter
73 Differential pressure related to blood removing catheter
74 Standard pressure related to oxygenator
75 Actual pressure related to oxygenator
76 Differential pressure related to oxygenator
77 Standard pressure related to blood feeding catheter
78 Actual pressure related to blood feeding catheter
79 Differential pressure related to blood feeding catheter
81 Pressure distribution
82 Standard head of centrifugal pump
83 Actual head of centrifugal pump
84 Circuit fixed pressure
85 Central venous pressure
331 Standard information related to blood removing catheter
332 Standard information related to oxygenator
333 Standard information related to blood feeding catheter
334 Standard information related to circulation circuit
431 Standard pressure calculation unit related to blood removing catheter
432 Standard pressure calculation unit related to oxygenator
433 Standard pressure calculation unit related to blood feeding catheter
441 Actual pressure calculation unit related to blood removing catheter
442 Actual pressure calculation unit related to oxygenator
443 Actual pressure calculation unit related to blood feeding catheter
461 Differential pressure calculation unit related to blood removing catheter
462 Differential pressure calculation unit related to oxygenator
463 Differential pressure calculation unit related to blood feeding catheter
P Patient

## Claims

1. An extracorporeal circulation management device (10) configured to manage an extracorporeal circulation device (1, 1A) extracorporeally circulating blood using a circulation circuit (1R), the extracorporeal circulation management device (10) configured to display an assumed flow rate, which is assumed in advance as an assumed value of a flow rate of the blood flowing inside the circulation circuit (1R) and which is calculated and determined by a standard flow rate calculation unit (47), and an actual flow rate, which is measured by a flow rate measurement unit (24) as an actually measured value of the flow rate of the blood flowing inside the circulation circuit (1R), on a display unit (16), and configured to display a standard pressure calculated by a standard pressure calculation unit (43) based on the assumed flow rate by referring to standard information, which represents a relation between the blood flow rate and a pressure loss calculated by an actual pressure calculation unit (44) occurring in a device provided in the circulation circuit (1R) and which is stored in a storage unit (30), and to further display an actual pressure related to said latter device, which is calculated based on an actual pressure measured by a pressure measurement unit (20) as an actually measured value of a pressure of the blood flowing inside the circulation circuit (1R) and the actual flow rate, on the display unit (16), wherein the standard pressure calculation unit (43), the actual pressure calculation unit (44) and the standard flow rate calculation unit (47) are included in a control unit (40) which in turn is included in the extracorporeal circulation management device (10).

2. The extracorporeal circulation management device (10) according to claim 1, wherein a differential flow rate representing a difference between the assumed flow rate and the actual flow rate and a differential pressure representing a difference between the standard pressure and the actual pressure related to the device are further displayed on the display unit (16).

3. The extracorporeal circulation management device (10) according to claim 2, wherein
the device includes a plurality of instrument elements,
the pressure measurement unit (20) includes a plurality of pressure sensors (21, 22, 23),
a plurality of the standard pressures calculated based on the assumed flow rate by referring to a plurality of pieces of the standard information each representing a relation between the blood flow rate and the pressure loss occurring in each of the plurality of instrument elements and stored in the storage unit (30), and the actual pressures related to the plurality of instrument elements, calculated based on a plurality of the actual pressures measured by the plurality of pressure sensors (21, 22, 23) and the actual flow rate, are displayed on the display unit (16), and a plurality of the differential pressures each representing a difference between each of the plurality of standard pressures and each of the actual pressures related to the plurality of instrument elements are displayed on the display unit (16).

4. The extracorporeal circulation management device (10) according to claim 3, wherein the plurality of instrument elements include:
a blood removing catheter (5) configured to be partially inserted into a patient (P) and guide the blood taken out from the patient (P);
a pump (3) configured to be provided on a downstream side of the blood removing catheter (5), takes out the blood from the patient through the blood removing catheter (5) and sends the blood to the downstream side;
an oxygenator (2) configured to be provided on the downstream side of the pump (3) and performs a gas exchange operation for the blood; and
a blood feeding catheter (6) configured to be provided on the downstream side of the oxygenator (2) and is partially inserted into the patient (P), and guide the blood that has passed through the oxygenator (2) to the patient (P),
the plurality of pressure sensors (21, 22, 23) include:
a first pressure sensor (21) configured to be provided in the circulation circuit (1R) at least between the blood removing catheter (5) and the pump (3) or between the pump (3) and the oxygenator (2); and
a second pressure sensor (22) configured to be provided in the circulation circuit (1R) between the oxygenator (2) and the blood feeding catheter (6),
the flow rate measurement unit (24) is a flow rate sensor configured to be provided in the circulation circuit (I R), and
the assumed flow rate,
the actual flow rate acquired by the flow rate sensor,
the differential flow rate,
the standard pressure related to the blood removing catheter (5) calculated based on the assumed flow rate by referring to the standard information related to the blood removing catheter (5) which represents a relation between the blood flow rate and the pressure loss occurring in the blood removing catheter (5) and is stored in the storage unit (30),
the standard pressure related to the oxygenator (2) calculated based on the assumed flow rate by referring to the standard information related to the oxygenator (2) which represents a relation between the blood flow rate and the pressure loss occurring in the oxygenator (2) and is stored in the storage unit (30),
the standard pressure related to the blood feeding catheter (6) calculated based on the assumed flow rate by referring to the standard information related to the blood feeding catheter (6) which represents a relation between the blood flow rate and the pressure loss occurring in the blood feeding catheter (6) and is stored in the storage unit (30),
the actual pressure related to the blood removing catheter (5) calculated based on the actual pressure measured by the first pressure sensor (21) and the actual flow rate,
the actual pressure related to the oxygenator (2) calculated based on the actual pressure measured by the first pressure sensor (21), the actual pressure measured by the second pressure sensor (22), and the actual flow rate,
the actual pressure related to the blood feeding catheter (6) calculated based on the actual pressure measured by the second pressure sensor (22) and the actual flow rate,
the differential pressure related to the blood removing catheter (5) which represents a difference between the standard pressure related to the blood removing catheter (5) and the actual pressure related to the blood removing catheter (5),
the differential pressure related to the oxygenator (2) which represents a difference between the standard pressure related to the oxygenator (2) and the actual pressure related to the oxygenator (2), and
the differential pressure related to the blood feeding catheter (6) which represents a difference between the standard pressure related to the blood feeding catheter (6) and the actual pressure related to the blood feeding catheter (6)
are all displayed on the display unit (16) simultaneously.

5. The extracorporeal circulation management device (10) according to claim 3, wherein the plurality of instrument elements include:
a blood removing catheter (5) configured to be partially inserted into a patient (P) and guides the blood taken out from the patient (P);
a pump (3) configured to be provided on a downstream side of the blood removing catheter (5), take out the blood from the patient (P) through the blood removing catheter (5), and send the blood to the downstream side;
an oxygenator (2) configured to be provided on the downstream side of the pump (3) and performs a gas exchange operation for the blood; and
a blood feeding catheter (6) configured to be provided on the downstream side of the oxygenator (2) and partially inserted into the patient (P), and guide the blood that has passed through the oxygenator (2) to the patient (P),
the plurality of pressure sensors (21, 22, 23) include:
a first pressure sensor (21) configured to be provided in the circulation circuit (1R) between the blood removing catheter (5) and the pump (3);
a second pressure sensor (22) configured to be provided in the circulation circuit (1R) between the pump (3) and the oxygenator (2); and
a third pressure sensor (23) configured to be provided in the circulation circuit (1R) between the oxygenator (2) and the blood feeding catheter (6),
the flow rate measurement unit (24) is a flow rate sensor configured to be provided in the circulation circuit (1R), and
the assumed flow rate,
the actual flow rate acquired by the flow rate sensor,
the differential flow rate,
the standard pressure related to the blood removing catheter (5) calculated based on the assumed flow rate by referring to the standard information related to the blood removing catheter (5) which represents a relation between the blood flow rate and the pressure loss occurring in the blood removing catheter (5) and is stored in the storage unit (30),
the standard pressure related to the oxygenator (2) calculated based on the assumed flow rate by referring to the standard information related to the oxygenator (2) which represents a relation between the blood flow rate and the pressure loss occurring in the oxygenator (2) and is stored in the storage unit (30),
the standard pressure related to the blood feeding catheter (6) calculated based on the assumed flow rate by referring to the standard information related to the blood feeding catheter (6) which represents a relation between the blood flow rate and the pressure loss occurring in the blood feeding catheter (6) and is stored in the storage unit (30),
the actual pressure related to the blood removing catheter (5) calculated based on the actual pressure measured by the first pressure sensor (21) and the actual flow rate,
the actual pressure related to the oxygenator (2) calculated based on the actual pressure measured by the second pressure sensor (22), the actual pressure measured by the third pressure sensor (23), and the actual flow rate,
the actual pressure related to the blood feeding catheter (6) calculated based on the actual pressure measured by the third pressure sensor (23) and the actual flow rate,
the differential pressure related to the blood removing catheter (5) which represents a difference between the standard pressure related to the blood removing catheter (5) and the actual pressure related to the blood removing catheter (5),
the differential pressure related to the oxygenator (2) which represents a difference between the standard pressure related to the oxygenator (2) and the actual pressure related to the oxygenator (2), and
the differential pressure related to the blood feeding catheter (6) which represents a difference between the standard pressure related to the blood feeding catheter (6) and the actual pressure related to the blood feeding catheter (6)
are all displayed on the display unit (16) simultaneously.

6. The extracorporeal circulation management device (10) according to any one of claims 2 to 5, wherein notification of a warning is provided when an absolute value of at least one of the differential flow rate and the differential pressure exceeds a predetermined value.

7. An extracorporeal circulation device (1, 1A) which extracorporeally circulates blood using a circulation circuit (1R), comprising:
a display unit (16) configured to display various types of information;
a blood removing catheter (5) configured to be partially inserted into a patient (P) and guide the blood taken out from the patient (P);
a pump (3) configured to be provided on a downstream side of the blood removing catheter (5), take out the blood from the patient (P) through the blood removing catheter (5), and send the blood to the downstream side;
an oxygenator (2) configured to be provided on the downstream side of the pump (3) and perform a gas exchange operation for the blood;
a blood feeding catheter (6) configured to be provided on the downstream side of the oxygenator (2), be partially inserted into the patient (P), and guide the blood that has passed through the oxygenator (2) to the patient (P); and
the extracorporeal circulation management device (10) according to any one of claims 1 to 6.

8. An extracorporeal circulation management program, executed by a computer of an extracorporeal circulation management device (10) configured to manage an extracorporeal circulation device (1, 1A) extracorporeally circulating blood using a circulation circuit (1R), the extracorporeal circulation management program causing the computer to execute
a step of displaying an assumed flow rate, which is assumed in advance as an assumed value of a flow rate of the blood flowing inside the circulation circuit (1R) and which is calculated and determined by a standard flow rate calculation unit (47), and an actual flow rate, which is measured by a flow rate measurement unit (24) as an actually measured value of the flow rate of the blood flowing inside the circulation circuit (1R), on a display unit (16), and displaying a standard pressure calculated by a standard pressure calculation unit (43) based on the assumed flow rate by referring to standard information, which represents a relation between the blood flow rate and a pressure loss calculated by an actual pressure calculation unit (44) occurring in a device provided in the circulation circuit (1R) and which is stored in a storage unit (30), and to further display an actual pressure related to said latter device, which is calculated based on an actual pressure measured by a pressure measurement unit (20) as an actually measured value of a pressure of the blood flowing inside the circulation circuit (1R) and the actual flow rate, on the display unit (16).

## Patentansprüche

1. Steuerungseinrichtung (10) für extrakorporale Zirkulation, die dafür konfiguriert ist, eine Einrichtung für extrakorporale Zirkulation (1, 1A) zu steuern, die unter Verwendung eines Zirkulationskreises (1R) extrakorporal Blut zirkulieren lässt, wobei die Steuerungseinrichtung (10) für extrakorporale Zirkulation dafür konfiguriert ist, eine angenommene Durchflussmenge, die im Voraus als ein angenommener Wert einer Durchflussmenge des Blutes, das innerhalb des Zirkulationskreises (1R) fließt, angenommen wird und die durch eine Normaldurchflussmengen-Berechnungseinheit (47) berechnet und bestimmt wird, und eine tatsächliche Durchflussmenge, die durch eine Durchflussmengen-Messeinheit (24) als ein tatsächlich gemessener Wert der Durchflussmenge des Blutes, das innerhalb des Zirkulationskreises (1R) fließt, gemessen wird, auf eine Anzeigeeinheit (16) anzuzeigen, und dafür konfiguriert ist, einen Normaldruck anzuzeigen, der durch eine Normaldruck-Berechnungseinheit (43) auf Grundlage der angenommenen Durchflussmenge durch Bezugnahme auf Standardinformationen berechnet wird, die eine Beziehung zwischen der Blutdurchflussmenge und einem durch eine Ist-Druck-Berechnungseinheit (44) berechneten Druckverlust, der in einer Einrichtung auftritt, die in dem Zirkulationskreis (1R) bereitgestellt wird, und der in einer Speichereinheit (30) gespeichert wird, darstellen, und ferner einen Ist-Druck in Zusammenhang mit der letzteren Einrichtung, der auf Grundlage eines Ist-Drucks, der durch eine Druckmesseinheit (20) als ein tatsächlich gemessener Wert eines Drucks des Blutes, das innerhalb des Zirkulationskreises (1R) strömt, gemessen wird, und der tatsächlichen Durchflussmenge berechnet wird, auf der Anzeigeeinheit (16) anzuzeigen, wobei die Normaldruck-Berechnungseinheit (43), die Ist-Druck-Berechnungseinheit (44) und die Normaldurchflussmengen-Berechnungseinheit (47) ist einer Steuereinheit (40) eingeschlossen sind, die wiederum in der Steuerungseinrichtung (10) für extrakorporale Zirkulation eingeschlossen ist.

2. Steuerungseinrichtung (10) für extrakorporale Zirkulation nach Anspruch 1, wobei eine Differenz-Durchflussmenge, die eine Differenz zwischen der angenommenen Durchflussmenge und der Tatsächlichen Durchflussmenge darstellt, und ein Differenzdruck, der eine Differenz zwischen dem Normaldruck und dem Ist-Druck in Zusammenhang mit der Einrichtung ferner auf der Anzeigeeinheit (16) angezeigt werden.

3. Steuerungseinrichtung (10) für extrakorporale Zirkulation nach Anspruch 2, wobei
die Einrichtung eine Vielzahl von Instrumentenelementen einschließt,
die Druckmesseinheit (20) eine Vielzahl von Drucksensoren (21, 22, 23) einschließt,
eine Vielzahl der Ist-Drücke, berechnet auf Grundlage der angenommenen Durchflussmenge durch Bezugnahme auf eine Vielzahl von Teilen der Standardinformationen, die jeweils eine Beziehung zwischen der Blutdurchflussmenge und dem Druckverlust, der in jedem von der Vielzahl von Instrumentenelementen auftritt und in der Speichereinheit (30) gespeichert wird, darstellen, und der Ist-Drücke in Zusammenhang mit der Vielzahl von Instrumentenelementen, berechnet auf Grundlage einer Vielzahl der Ist-Drücke, die durch die Vielzahl von Drucksensoren (21, 22, 23) gemessen werden, und der tatsächlichen Durchflussmenge, auf der Anzeigeeinheit (16) angezeigt werden und eine Vielzahl der Differenzdrücke, die jeweils eine Differenz zwischen jedem von der Vielzahl von Normaldrücken und jedem von der Ist-Drücke in Zusammenhang mit der Vielzahl von Instrumentenelementen darstellen, auf der Anzeigeeinheit (16) angezeigt werden.

4. Steuerungseinrichtung (10) für extrakorporale Zirkulation nach Anspruch 3, wobei
die Vielzahl von Instrumentenelementen Folgendes einschließt:
einen Blutentnahmekatheter (5), der dafür konfiguriert ist, teilweise in einen Patienten (P) eingeführt zu werden und das Blut, das entnommen wird, aus dem Patienten (P) zu leiten,
eine Pumpe (3), die dafür konfiguriert ist, auf einer Stromabwärtsseite des Blutentnahmekatheters (5) bereitgestellt zu werden, das Blut aus dem Patienten durch den Blutentnahmekatheter (5) zu entnehmen und das Blut zu der Stromabwärtsseite zu schicken,
einen Oxygenator (2), der dafür konfiguriert ist, auf der Stromabwärtsseite der Pumpe (3) bereitgestellt zu werden und einen Gasaustauschvorgang für das Blut durchzuführen, und
einen Blutzufuhrkatheter (6), der dafür konfiguriert ist, auf der Stromabwärtsseite des Oxygenators (2) bereitgestellt zu werden und teilweise in den Patienten (P) eingeführt zu werden und das Blut, das durch den Oxygenator (2) hindurchgegangen ist, zu dem Patienten (P) zu leiten,
wobei die Vielzahl von Drucksensoren (21, 22, 23) Folgendes einschließt:
einen ersten Drucksensor (21), der dafür konfiguriert ist, in dem Zirkulationskreis (1R) zumindest zwischen dem Blutentnahmekatheter (5) und der Pumpe (3) oder zwischen der Pumpe (3) und dem Oxygenator (2) bereitgestellt zu werden, und
einen zweiten Drucksensor (22), der dafür konfiguriert ist, in dem Zirkulationskreis (1R) zwischen dem Oxygenator (2) und dem Blutzufuhrkatheter (6) bereitgestellt zu werden,
wobei die Durchflussmengen-Messeinheit (24) ein Durchflussmengensensor ist, der dafür konfiguriert ist, in dem Zirkulationskreis (1R) bereitgestellt zu werden, und
die angenommene Durchflussmenge,
die tatsächliche Durchflussmenge, die durch den Durchflussmengensensor erfasst wird,
die Differenz-Durchflussmenge,
der Normaldruck in Zusammenhang mit dem Blutentnahmekatheter (5), berechnet auf Grundlage der angenommenen Durchflussmenge durch Bezugnahme auf die Standardinformationen in Zusammenhang mit dem Blutentnahmekatheter (5), die eine Beziehung zwischen der Blutdurchflussmenge und dem Druckverlust, der in dem Blutentnahmekatheter (5) auftritt und in der Speichereinheit (30) gespeichert wird, darstellen,
der Normaldruck in Zusammenhang mit dem Oxygenator (2), berechnet auf Grundlage der angenommenen Durchflussmenge durch Bezugnahme auf die Standardinformationen in Zusammenhang mit dem Oxygenator (2), die eine Beziehung zwischen der Blutdurchflussmenge und dem Druckverlust, der in dem Oxygenator (2) auftritt und in der Speichereinheit (30) gespeichert wird, darstellen,
der Normaldruck in Zusammenhang mit dem Blutzufuhrkatheter (6), berechnet auf Grundlage der angenommenen Durchflussmenge durch Bezugnahme auf die Standardinformationen in Zusammenhang mit dem Blutzufuhrkatheter (6), die eine Beziehung zwischen der Blutdurchflussmenge und dem Druckverlust, der in dem Blutzufuhrkatheter (6) auftritt und in der Speichereinheit (30) gespeichert wird, darstellen,
der Ist-Druck in Zusammenhang mit dem Blutentnahmekatheter (5), berechnet auf Grundlage des Ist-Drucks, der durch den ersten Drucksensor (21) gemessen wird, und der tatsächlichen Durchflussmenge,
der Ist-Druck in Zusammenhang mit dem Oxygenator (2), berechnet auf Grundlage des Ist-Drucks, der durch den ersten Drucksensor (21) gemessen wird, des Ist-Drucks, der durch den zweiten Drucksensor (22) gemessen wird, und der tatsächlichen Durchflussmenge,
der Ist-Druck in Zusammenhang mit dem Blutzufuhrkatheter (6), berechnet auf Grundlage des Ist-Drucks, der durch den zweiten Drucksensor (22) gemessen wird, und der tatsächlichen Durchflussmenge, der Differenzdruck in Zusammenhang mit dem Blutentnahmekatheter (5), der eine Differenz zwischen dem Normaldruck in Zusammenhang mit dem Blutentnahmekatheter (5) und dem Ist-Druck in Zusammenhang mit dem Blutentnahmekatheter (5) darstellt,
der Differenzdruck in Zusammenhang mit dem Oxygenator (2), der eine Differenz zwischen dem Normaldruck in Zusammenhang mit dem Oxygenator (2) dem Ist-Druck in Zusammenhang mit dem Oxygenator (2) darstellt, und der Differenzdruck in Zusammenhang mit dem Blutzufuhrkatheter (6), der eine Differenz zwischen dem Normaldruck in Zusammenhang mit dem Blutzufuhrkatheter (6) und dem Ist-Druck in Zusammenhang mit dem Blutzufuhrkatheter (6) darstellt,
alle gleichzeitig auf der Anzeigeeinheit (16) angezeigt werden.

5. Steuerungseinrichtung (10) für extrakorporale Zirkulation nach Anspruch 3, wobei
die Vielzahl von Instrumentenelementen Folgendes einschließt:
einen Blutentnahmekatheter (5), der dafür konfiguriert ist, teilweise in einen Patienten (P) eingeführt zu werden und das Blut, das entnommen wird, aus dem Patienten (P) zu leiten,
eine Pumpe (3), die dafür konfiguriert ist, auf einer Stromabwärtsseite des Blutentnahmekatheters (5) bereitgestellt zu werden, das Blut aus dem Patienten (P) durch den Blutentnahmekatheter (5) entnimmt und das Blut zu der Stromabwärtsseite schickt,
einen Oxygenator (2), der dafür konfiguriert ist, auf der Stromabwärtsseite der Pumpe (3) bereitgestellt zu werden und einen Gasaustauschvorgang für das Blut durchzuführen, und
einen Blutzufuhrkatheter (6), der dafür konfiguriert ist, auf der Stromabwärtsseite des Oxygenators (2) bereitgestellt zu werden, und teilweise in den Patienten (P) eingeführt wird, und das Blut, das durch den Oxygenator (2) hindurchgegangen ist, zu dem Patienten (P) zu leiten,
wobei die Vielzahl von Drucksensoren (21, 22, 23) Folgendes einschließt:
einen ersten Drucksensor (21), der dafür konfiguriert ist, in dem Zirkulationskreis (1R) zwischen dem Blutentnahmekatheter (5) und der Pumpe (3) bereitgestellt zu werden,
einen zweiten Drucksensor (22), der dafür konfiguriert ist, in dem Zirkulationskreis (1R) zwischen der Pumpe (3) und dem Oxygenator (2) bereitgestellt zu werden, und
einen dritten Drucksensor (23), der dafür konfiguriert ist, in dem Zirkulationskreis (1R) zwischen dem Oxygenator (2) und dem Blutzufuhrkatheter (6) bereitgestellt zu werden,
wobei die Durchflussmengen-Messeinheit (24) ein Durchflussmengensensor ist, der dafür konfiguriert ist, in dem Zirkulationskreis (1R) bereitgestellt zu werden, und
die angenommene Durchflussmenge,
die tatsächliche Durchflussmenge, die durch den Durchflussmengensensor erfasst wird,
die Differenz-Durchflussmenge,
der Normaldruck in Zusammenhang mit dem Blutentnahmekatheter (5), berechnet auf Grundlage der angenommenen Durchflussmenge durch Bezugnahme auf die Standardinformationen in Zusammenhang mit dem Blutentnahmekatheter (5), die eine Beziehung zwischen der Blutdurchflussmenge und dem Druckverlust, der in dem Blutentnahmekatheter (5) auftritt und in der Speichereinheit (30) gespeichert wird, darstellen,
der Normaldruck in Zusammenhang mit dem Oxygenator (2), berechnet auf Grundlage der angenommenen Durchflussmenge durch Bezugnahme auf die Standardinformationen in Zusammenhang mit dem Oxygenator (2), die eine Beziehung zwischen der Blutdurchflussmenge und dem Druckverlust, der in dem Oxygenator (2) auftritt und in der Speichereinheit (30) gespeichert wird, darstellen,
der Normaldruck in Zusammenhang mit dem Blutzufuhrkatheter (6), berechnet auf Grundlage der angenommenen Durchflussmenge durch Bezugnahme auf die Standardinformationen in Zusammenhang mit dem Blutzufuhrkatheter (6), die eine Beziehung zwischen der Blutdurchflussmenge und dem Druckverlust, der in dem Blutzufuhrkatheter (6) auftritt und in der Speichereinheit (30) gespeichert wird, darstellen,
der Ist-Druck in Zusammenhang mit dem Blutentnahmekatheter (5), berechnet auf Grundlage des Ist-Drucks, der durch den ersten Drucksensor (21) gemessen wird, und der tatsächlichen Durchflussmenge,
der Ist-Druck in Zusammenhang mit dem Oxygenator (2), berechnet auf Grundlage des Ist-Drucks, der durch den zweiten Drucksensor (22) gemessen wird, des Ist-Drucks, der durch den dritten Drucksensor (23) gemessen wird, und der tatsächlichen Durchflussmenge,
der Ist-Druck in Zusammenhang mit dem Blutzufuhrkatheter (6), berechnet auf Grundlage des Ist-Drucks, der durch den dritten Drucksensor (23) gemessen wird, und der tatsächlichen Durchflussmenge,
der Differenzdruck in Zusammenhang mit dem Blutentnahmekatheter (5), der eine Differenz zwischen dem Normaldruck in Zusammenhang mit dem Blutentnahmekatheter (5) und dem Ist-Druck in Zusammenhang mit dem Blutentnahmekatheter (5) darstellt,
der Differenzdruck in Zusammenhang mit dem Oxygenator (2), der eine Differenz zwischen dem Normaldruck in Zusammenhang mit dem Oxygenator (2) dem Ist-Druck in Zusammenhang mit dem Oxygenator (2) darstellt, und
der Differenzdruck in Zusammenhang mit dem Blutzufuhrkatheter (6), der eine Differenz zwischen dem Normaldruck in Zusammenhang mit dem Blutzufuhrkatheter (6) und dem Ist-Druck in Zusammenhang mit dem Blutzufuhrkatheter (6) darstellt,
alle gleichzeitig auf der Anzeigeeinheit (16) angezeigt werden.

6. Steuerungseinrichtung (10) für extrakorporale Zirkulation nach einem der Anspruche 2 bis 5, wobei eine Benachrichtigung einer Warnung bereitgestellt wird, wenn ein absoluter Wert mindestens eines von der Differenz-Durchflussmenge und dem Differenzdruck einen vorbestimmten Wert überschreitet.

7. Einrichtung für extrakorporale Zirkulation (1, 1A), die unter Verwendung eines Zirkulationskreises (1R) extrakorporal Blut zirkulieren lässt, wobei sie Folgendes umfasst:
eine Anzeigeeinheit (16), die dafür konfiguriert ist, verschiedene Arten von Informationen anzuzeigen,
einen Blutentnahmekatheter (5), der dafür konfiguriert ist, teilweise in einen Patienten (P) eingeführt zu werden und das Blut, das entnommen wird, aus dem Patienten (P) zu leiten,
eine Pumpe (3), die dafür konfiguriert ist, auf einer Stromabwärtsseite des Blutentnahmekatheters (5) bereitgestellt zu werden, das Blut aus dem Patienten durch den Blutentnahmekatheter (5) zu entnehmen und das Blut zu der Stromabwärtsseite zu schicken,
einen Oxygenator (2), der dafür konfiguriert ist, auf der Stromabwärtsseite der Pumpe (3) bereitgestellt zu werden und einen Gasaustauschvorgang für das Blut durchzuführen,
einen Blutzufuhrkatheter (6), der dafür konfiguriert ist, auf der Stromabwärtsseite des Oxygenators (2) bereitgestellt zu werden, teilweise in den Patienten (P) eingeführt zu werden und das Blut, das durch den Oxygenator (2) hindurchgegangen ist, zu dem Patienten (P) zu leiten, und
die Steuerungseinrichtung (10) für extrakorporale Zirkulation nach einem der Ansprüche 1 bis 6.

8. Steuerungsprogramm für extrakorporale Zirkulation, ausgeführt durch einen Rechner einer Steuerungseinrichtung (10) für extrakorporale Zirkulation, die dafür konfiguriert ist, eine Einrichtung für extrakorporale Zirkulation (1, 1A) zu steuern, die unter Verwendung eines Zirkulationskreises (1R) extrakorporal Blut zirkulieren lässt, wobei das Steuerungsprogramm für extrakorporale Zirkulation veranlasst, dass der Rechner Folgendes ausführt:
einen Schritt des Anzeigens einer angenommenen Durchflussmenge, die im Voraus als ein angenommener Wert einer Durchflussmenge des Blutes, das innerhalb des Zirkulationskreises (1R) fließt, angenommen wird und die durch eine Normaldurchflussmengen-Berechnungseinheit (47) berechnet und bestimmt wird, und einer tatsächlichen Durchflussmenge, die durch eine Durchflussmengen-Messeinheit (24) als ein tatsächlich gemessener Wert der Durchflussmenge des Blutes, das innerhalb des Zirkulationskreises (1R) fließt, gemessen wird, auf eine Anzeigeeinheit (16), und des Anzeigens eines Normaldrucks, der durch eine Normaldruck-Berechnungseinheit (43) auf Grundlage der angenommenen Durchflussmenge durch Bezugnahme auf Standardinformationen berechnet wird, die eine Beziehung zwischen der Blutdurchflussmenge und einem durch eine Ist-Druck-Berechnungseinheit (44) berechneten Druckverlust, der in einer Einrichtung auftritt, die in dem Zirkulationskreis (1R) bereitgestellt wird, und der in einer Speichereinheit (30) gespeichert wird, darstellen, und ferner einen Ist-Druck in Zusammenhang mit der letzteren Einrichtung, der auf Grundlage eines Ist-Drucks, der durch eine Druckmesseinheit (20) als ein tatsächlich gemessener Wert eines Drucks des Blutes, das innerhalb des Zirkulationskreises (1R) strömt, gemessen wird, und der tatsächlichen Durchflussmenge berechnet wird, auf der Anzeigeeinheit (16) anzuzeigen.

## Revendications

1. Dispositif de gestion de circulation extracorporelle (10) configuré pour gérer un dispositif de circulation extracorporelle (1, 1A) faisant circuler du sang de manière extracorporelle à l'aide d'un circuit de circulation (1R), le dispositif de gestion de circulation extracorporelle (10) étant configuré pour afficher un débit présumé, qui est présumé à l'avance comme une valeur présumée d'un débit du sang circulant à l'intérieur du circuit de circulation (1R) et qui est calculé et déterminé par une unité de calcul de débit standard (47), et un débit réel, qui est mesuré par une unité de mesure de débit (24) comme une valeur réellement mesurée du débit du sang circulant à l'intérieur du circuit de circulation (1R), sur une unité d'affichage (16), et configuré pour afficher une pression standard calculée par une unité de calcul de pression standard (43) sur la base du débit présumé en se référant à des informations standard, qui représentent une relation entre le débit sanguin et une perte de pression calculée par une unité de calcul de pression réelle (44) survenant dans un dispositif ménagé dans le circuit de circulation (1R) et qui sont stockées dans une unité de stockage (30), et pour afficher en outre une pression réelle relative audit dispositif, qui est calculée sur la base d'une pression réelle mesurée par une unité de mesure de pression (20) comme une valeur réellement mesurée d'une pression du sang circulant à l'intérieur du circuit de circulation (1R) et du débit réel, sur l'unité d'affichage (16), dans lequel l'unité de calcul de pression standard (43), l'unité de calcul de pression réelle (44) et l'unité de calcul de débit standard (47) sont incluses dans une unité de commande (40) qui à son tour est incluse dans le dispositif de gestion de circulation extracorporelle (10).

2. Dispositif de gestion de circulation extracorporelle (10) selon la revendication 1, dans lequel un débit différentiel représentant une différence entre le débit présumé et le débit réel et une pression différentielle représentant une différence entre la pression standard et la pression réelle relative au dispositif sont en outre affichés sur l'unité d'affichage (16).

3. Dispositif de gestion de circulation extracorporelle (10) selon la revendication 2, dans lequel le dispositif inclut une pluralité d'éléments d'instrument, l'unité de mesure de pression (20) inclut une pluralité de capteurs de pression (21, 22, 23),
une pluralité des pressions standard calculées sur la base du débit présumé en se référant à une pluralité de fragments des informations standard représentant chacune une relation entre le débit sanguin et la perte de pression survenant dans chacun de la pluralité d'éléments d'instrument et stockées dans l'unité de stockage (30), et les pressions réelles relatives à la pluralité d'éléments d'instrument, calculées sur la base d'une pluralité des pressions réelles mesurées par la pluralité de capteurs de pression (21, 22, 23) et du débit réel, sont affichées sur l'unité d'affichage (16), et une pluralité des pressions différentielles représentant chacune une différence entre chacune de la pluralité de pressions standard et chacune des pressions réelles relatives à la pluralité d'éléments d'instrument sont affichées sur l'unité d'affichage (16).

4. Dispositif de gestion de circulation extracorporelle (10) selon la revendication 3, dans lequel la pluralité d'éléments d'instrument incluent :
un cathéter d'extraction de sang (5) configuré pour être partiellement inséré dans un patient (P) et guider le sang prélevé chez le patient (P) ;
une pompe (3) configurée pour être ménagée sur un côté aval du cathéter d'extraction de sang (5), qui prélève le sang chez le patient à travers le cathéter d'extraction de sang (5) et envoie le sang vers le côté aval ;
un oxygénateur (2) configuré pour être ménagé sur le côté aval de la pompe (3) et qui réalise une opération d'échange gazeux pour le sang ; et
un cathéter d'apport de sang (6) configuré pour être ménagé sur le côté aval de l'oxygénateur (2) et qui est partiellement inséré dans le patient (P), et guider le sang qui a traversé l'oxygénateur (2) jusqu'au patient (P),
la pluralité de capteurs de pression (21, 22, 23) incluent :
un premier capteur de pression (21) configuré pour être ménagé dans le circuit de circulation (1R) au moins entre le cathéter d'extraction de sang (5) et la pompe (3) ou entre la pompe (3) et l'oxygénateur (2) ; et
un deuxième capteur de pression (22) configuré pour être ménagé dans le circuit de circulation (1R) entre l'oxygénateur (2) et le cathéter d'apport de sang (6),
l'unité de mesure de débit (24) est un capteur de débit configuré pour être ménagé dans le circuit de circulation (1R), et
le débit présumé,
le débit réel acquis par le capteur de débit,
le débit différentiel,
la pression standard relative au cathéter d'extraction de sang (5) calculée sur la base du débit présumé en se référant aux informations standard relatives au cathéter d'extraction de sang (5) qui représentent une relation entre le débit sanguin et la perte de pression survenant dans le cathéter d'extraction de sang (5) et sont stockées dans l'unité de stockage (30),
la pression standard relative à l'oxygénateur (2) calculée sur la base du débit présumé en se référant aux informations standard relatives à l'oxygénateur (2) qui représentent une relation entre le débit sanguin et la perte de pression survenant dans l'oxygénateur (2) et sont stockées dans l'unité de stockage (30),
la pression standard relative au cathéter d'apport de sang (6) calculée sur la base du débit présumé en se référant aux informations standard relatives au cathéter d'apport de sang (6) qui représentent une relation entre le débit sanguin et la perte de pression survenant dans le cathéter d'apport de sang (6) et sont stockées dans l'unité de stockage (30),
la pression réelle relative au cathéter d'extraction de sang (5) calculée sur la base de la pression réelle mesurée par le premier capteur de pression (21) et du débit réel, la pression réelle relative à l'oxygénateur (2) calculée sur la base de la pression réelle mesurée par le premier capteur de pression (21), de la pression réelle mesurée par le deuxième capteur de pression (22), et du débit réel, la pression réelle relative au cathéter d'apport de sang (6) calculée sur la base de la pression réelle mesurée par le deuxième capteur de pression (22) et du débit réel,
la pression différentielle relative au cathéter d'extraction de sang (5) qui représente une différence entre la pression standard relative au cathéter d'extraction de sang (5) et la pression réelle relative au cathéter d'extraction de sang (5),
la pression différentielle relative à l'oxygénateur (2) qui représente une différence entre la pression standard relative à l'oxygénateur (2) et la pression réelle relative à l'oxygénateur (2), et
la pression différentielle relative au cathéter d'apport de sang (6) qui représente une différence entre la pression standard relative au cathéter d'apport de sang (6) et la pression réelle relative au cathéter d'apport de sang (6) sont tous affichés sur l'unité d'affichage (16) simultanément.

5. Dispositif de gestion de circulation extracorporelle (10) selon la revendication 3, dans lequel la pluralité d'éléments d'instrument incluent :
un cathéter d'extraction de sang (5) configuré pour être partiellement inséré dans un patient (P) et qui guide le sang prélevé chez le patient (P) ;
une pompe (3) configurée pour être ménagée sur un côté aval du cathéter d'extraction de sang (5), prélever le sang chez le patient à travers le cathéter d'extraction de sang (5) et envoyer le sang vers le côté aval ;
un oxygénateur (2) configuré pour être ménagé sur le côté aval de la pompe (3) et qui réalise une opération d'échange gazeux pour le sang ; et
un cathéter d'apport de sang (6) configuré pour être ménagé sur le côté aval de l'oxygénateur (2) et partiellement inséré dans le patient (P), et guider le sang qui a traversé l'oxygénateur (2) jusqu'au patient (P),
la pluralité de capteurs de pression (21, 22, 23) incluent :
un premier capteur de pression (21) configuré pour être ménagé dans le circuit de circulation (1R) entre le cathéter d'extraction de sang (5) et la pompe (3) ;
un deuxième capteur de pression (22) configuré pour être ménagé dans le circuit de circulation (1R) entre la pompe (3) et l'oxygénateur (2) ; et
un troisième capteur de pression (23) configuré pour être ménagé dans le circuit de circulation (1R) entre l'oxygénateur (2) et le cathéter d'apport de sang (6), l'unité de mesure de débit (24) est un capteur de débit configuré pour être ménagé dans le circuit de circulation (1R), et
le débit présumé,
le débit réel acquis par le capteur de débit,
le débit différentiel,
la pression standard relative au cathéter d'extraction de sang (5) calculée sur la base du débit présumé en se référant aux informations standard relatives au cathéter d'extraction de sang (5) qui représentent une relation entre le débit sanguin et la perte de pression survenant dans le cathéter d'extraction de sang (5) et sont stockées dans l'unité de stockage (30),
la pression standard relative à l'oxygénateur (2) calculée sur la base du débit présumé en se référant aux informations standard relatives à l'oxygénateur (2) qui représentent une relation entre le débit sanguin et la perte de pression survenant dans l'oxygénateur (2) et sont stockées dans l'unité de stockage (30),
la pression standard relative au cathéter d'apport de sang (6) calculée sur la base du débit présumé en se référant aux informations standard relatives au cathéter d'apport de sang (6) qui représentent une relation entre le débit sanguin et la perte de pression survenant dans le cathéter d'apport de sang (6) et sont stockées dans l'unité de stockage (30),
la pression réelle relative au cathéter d'extraction de sang (5) calculée sur la base de la pression réelle mesurée par le premier capteur de pression (21) et du débit réel,
la pression réelle relative à l'oxygénateur (2) calculée sur la base de la pression réelle mesurée par le deuxième capteur de pression (22), de la pression réelle mesurée par le troisième capteur de pression (23), et du débit réel,
la pression réelle relative au cathéter d'apport de sang (6) calculée sur la base de la pression réelle mesurée par le troisième capteur de pression (23) et du débit réel,
la pression différentielle relative au cathéter d'extraction de sang (5) qui représente une différence entre la pression standard relative au cathéter d'extraction de sang (5) et la pression réelle relative au cathéter d'extraction de sang (5),
la pression différentielle relative à l'oxygénateur (2) qui représente une différence entre la pression standard relative à l'oxygénateur (2) et la pression réelle relative à l'oxygénateur (2), et
la pression différentielle relative au cathéter d'apport de sang (6) qui représente une différence entre la pression standard relative au cathéter d'apport de sang (6) et la pression réelle relative au cathéter d'apport de sang (6) sont tous affichés sur l'unité d'affichage (16) simultanément.

6. Dispositif de gestion de circulation extracorporelle (10) selon l'une quelconque des revendications 2 à 5, dans lequel une notification d'un avertissement est fournie lorsqu'une valeur absolue d'au moins l'un parmi le débit différentiel et la pression différentielle dépasse une valeur prédéterminée.

7. Dispositif de circulation extracorporelle (1, 1A) qui fait circuler du sang de manière extracorporelle à l'aide d'un circuit de circulation (1R), comprenant :
une unité d'affichage (16) configurée pour afficher divers types d'informations ;
un cathéter d'extraction de sang (5) configuré pour être partiellement inséré dans un patient (P) et guider le sang prélevé chez le patient (P) ;
une pompe (3) configurée pour être ménagée sur un côté aval du cathéter d'extraction de sang (5), prélever le sang chez le patient à travers le cathéter d'extraction de sang (5) et envoyer le sang vers le côté aval ;
un oxygénateur (2) configuré pour être ménagé sur le côté aval de la pompe (3) et réaliser une opération d'échange gazeux pour le sang ;
un cathéter d'apport de sang (6) configuré pour être ménagé sur le côté aval de l'oxygénateur (2), être partiellement inséré dans le patient (P), et guider le sang qui a traversé l'oxygénateur (2) jusqu'au patient (P) ; et
le dispositif de gestion de circulation extracorporelle (10) selon l'une quelconque des revendications 1 à 6.

8. Programme de gestion de circulation extracorporelle, exécuté par un ordinateur d'un dispositif de gestion de circulation extracorporelle (10) configuré pour gérer un dispositif de circulation extracorporelle (1, 1A) faisant circuler du sang de manière extracorporelle à l'aide d'un circuit de circulation (1R), le programme de gestion de circulation extracorporelle amenant l'ordinateur à exécuter
une étape d'affichage d'un débit présumé, qui est présumé à l'avance comme une valeur présumée d'un débit du sang circulant à l'intérieur du circuit de circulation (1R) et qui est calculé et déterminé par une unité de calcul de débit standard (47), et d'un débit réel, qui est mesuré par une unité de mesure de débit (24) comme une valeur réellement mesurée du débit du sang circulant à l'intérieur du circuit de circulation (1R), sur une unité d'affichage (16), et d'affichage d'une pression standard calculée par une unité de calcul de pression standard (43) sur la base du débit présumé en se référant à des informations standard, qui représentent une relation entre le débit sanguin et une perte de pression calculée par une unité de calcul de pression réelle (44) survenant dans un dispositif ménagé dans le circuit de circulation (1R) et qui sont stockées dans une unité de stockage (30), et pour afficher en outre une pression réelle relative audit dispositif, qui est calculée sur la base d'une pression réelle mesurée par une unité de mesure de pression (20) comme une valeur réellement mesurée d'une pression du sang circulant à l'intérieur du circuit de circulation (1R) et du débit réel, sur l'unité d'affichage (16).
